(19) Europäisches Patentamt
European Patent Office
Office européen des brevets

(11) **EP 1 210 052 B1**

(12) **EUROPEAN PATENT SPECIFICATION**

(45) Date of publication and mention
of the grant of the patent:
**23.01.2008 Bulletin 2008/04**

(51) Int Cl.:
*A61J 7/04* *(2006.01)*

(21) Application number: **00949028.5**

(22) Date of filing: **21.07.2000**

(86) International application number:
**PCT/CA2000/000847**

(87) International publication number:
**WO 2001/008106 (01.02.2001 Gazette 2001/05)**

(54) **PACKAGE WITH INTEGRATED CIRCUIT CHIP EMBEDDED THEREIN AND SYSTEM FOR USING SAME**

BEHÄLTER MIT EINGEBETTETEN INTEGRIERTEN SCHALTUNGSCHIP UND SYSTEM ZU IHRER ANWENDUNG

BOITIER A MICROCIRCUIT INTEGRE NOYE DANS CELUI-CI ET SYSTEME D'UTILISATION

(84) Designated Contracting States:
**AT BE CH CY DE DK ES FI FR GB GR IE IT LI LU MC NL PT SE**

(30) Priority: **23.07.1999 US 359322**

(43) Date of publication of application:
**05.06.2002 Bulletin 2002/23**

(73) Proprietors:
• **Momich, Robert**
**Hamilton,**
**Ontario L8X 6L4 (CA)**
• **Infuso, Michael E.**
**Toronto,**
**Ontario M9A 4B3 (CA)**

(72) Inventors:
• **Momich, Robert**
**Hamilton,**
**Ontario L8X 6L4 (CA)**

• **Infuso, Michael E.**
**Toronto,**
**Ontario M9A 4B3 (CA)**

(74) Representative: **Langley, Peter James**
**Origin Limited,**
**52 Muswell Hill Road**
**London N10 3JR (GB)**

(56) References cited:
WO-A-99/17218        US-A- 4 504 153
US-A- 4 616 316       US-A- 4 695 954
US-A- 4 831 562       US-A- 5 181 189
US-A- 5 495 961       US-A- 5 802 015
US-A- 5 805 051       US-A- 5 812 064
US-A- 5 852 590

## Description

### FIELD OF THE INVENTION

This invention relates to an interactive reminder device to prompt a patient to take medication.

### BACKGROUND OF THE INVENTION

The correct administration of medications whether by an individual or a health care professional is a growing concern. In particular it is a concern where the individual takes multiple medications. Further, it is a concern as the individual gets older to ensure that he/she is taking their medication as prescribed. It is also a concern to ensure that the individual gives each health care professional a full history of medications as well as related problems.

Typically when a physician prescribes a particular medication, the physician has very little control over whether the patient actually takes the medication as prescribed. Rather, the physician must rely on the patient to take the medication as prescribed. The risk of a patient taking their medication incorrectly is increased with the number of medications. Studies have shown that in the United States the hospitalization of patients due to the incorrect administration of prescriptions drugs has increased in recent years. In some instances the incorrect use of the drug is fatal.

Other studies have shown that 60% of women on the birth control pill have forgotten to take the pill everyday and as few as 20% of women actually take the pill at the same time everyday as prescribed. The result of this is that most unplanned pregnancies occur because women do not take their birth control pill regularly. One way the government is trying to address this issue is to require more comprehensive information on the drug labels. However, more effective labelling is only a partial solution since people will still forget to take the medication either at all or at the correct time.

There are concerns related to the administration of medication that need to be considered. For example some concerns are: how to ensure that the individual does not forget to take the medication at the prescribed times or at the prescribed intervals; how to ensure that the individual takes neither too few nor too many medications; and how to ensure that the individual takes the right medication or that they heed the warnings or other contra-indicators associated with the medication. In addition, it would be advantageous if the physician had a mechanism to confirm when in fact the medications were taken. This is of particular importance when the physician is trying to make minor adjustments to the medication.

In addition to these concerns, when the medication is administered by a health care professional in a hospital or other institution there are other concerns that need to be considered. For example other concerns are: how to ensure there is continuity with multiple staff for one individual; how to ensure that the medication is administered to the right individual; and how to ensure that there is a complete log of all of the medications given to a specific individual.

As well with the advent of internet commerce there are concerns about regulating the sale of pharmaceutical products on-line. The concerns regarding internet pharmaceutical sales were sparked by recent reports of an estimated 400 Web sites that are allegedly filling prescriptions without valid authorizations from physicians or licensed pharmacists. Apparently prescriptions can be filled via these sites without the buyer having ever visited a doctor. Accordingly there is clearly a need to regulate the sale of pharmaceuticals on-line. There are also concerns from the perspective of the pharmaceutical merchants because on-line sales without some kind of verification process may lead to an increased exposure to liability. Documentation and proof of compliance with regulations will be of increasing concern as legal challenges to on-line sales steadily increase over the next few years. Consumers will undoubtedly seek opportunities to pass liability onto manufacturers and on-line retailers, even if the abuse starts with matters that are their responsibility.

Further, when considering the on-line purchase of pharmaceutical products, it is clear that the normal checks and balances used when purchasing at a retail pharmacy will need to be adapted for the on-line environment. It is important to have checks and balances in the sale of pharmaceutical products since many of the products are controlled substances and since there are serious consequences of misuse. It is important when selling pharmaceutical products to confirm that a licensed, authorized medical professional has issued the prescription. Protocols are in place to ensure that there are adequate checks and balances in place when the sale takes place at a retail pharmacy however this is not currently the case with regard to on-line purchases. Accordingly with on-line purchases it will be easier for a person to obtain prescription medicine without in fact having a prescription or obtaining a repeat without authorization. Currently some on-line pharmacies require the purchaser to provide a copy of the prescription by mail or verify the prescription with the physician. Clearly these solutions are time consuming and, rather than simplifying the process, the purchase may in fact complicate it. Currently there are no efficient mechanisms for observing the verification standards maintained by an on-line site for the purchase of pharmaceutical products.

A number of devices have been suggested to try to address at least some of these problems. For example, a number of devices are used as reminder units for specific medications containers. For example US patent 4,616,316 issued to Hanpeter et al. shows a monitoring device attached to a blister pack having conductive traces thereon such that when a trace is broken the monitoring device records the time thereof. The monitoring device includes a power source. US patent 5,181,189 issued January 19, 1993 to Hafner shows a signalling device that is adapted to be attached to an individual medication. The device signals when the medication is to be taken but it does not record if it has been taken. US patent 5,495,961 issued March 5, 1996 to Maestre shows a programmable medication alarm device designed to be attached to a medicine bottle or dispenser. Similarly, US patent 4,504,153 issued March 12, 1985 to Schollmeyer et al. shows a prompting device that is attached to an individual medication container. The Schollmeyer device may record when the cap was opened. There are a number of shortcomings with regard to these devices. In particular, all of these devices include a power source with the device and therefore with the medication and accordingly these devices would be expensive to operate. Further, these devices do not have a method of providing contra-indications or handling multiple medications.

Alternatively, more comprehensive devices have been suggested. For example US patent 4,831,562 issued May 16, 1989 to McIntosh et al. shows a reminder device that includes individual compartments for each different medication. The device is programmed to provide a reminder signal and provide a display to indicate that a medication should be taken. Similarly US patent 5,805,051 issued September 8, 1998 to Hermann et al. shows an interactive medication reminder/dispenser device with a plurality of compartments for different medications. The device provides a visual and audio signal indicative of a time to take at least one medication. This device may also include a weighing mechanism or an optical system which enables the device to determine if a pill has actually been removed. Alternatively the user presses a key to indicate that the medication has been taken. Both devices could also include information with regard to medication incompatibilities. Each of these devices has a power source attached thereto. There are a number of shortcomings with regard to these devices. In particular by emptying the medication into another container the user risks confusing the medication by inadvertently putting it into the wrong compartment. Further, information from the medications is read into the device but no information with regard to the use of the medication is written back to the medication container. Therefore, no information with regard to use of the medication is attached to the medication container.

Other devices have been suggested which have specific functions with regard to the safe use of medications. For example US patent 5,812,064 issued September 22, 1998 to Barbour shows a device that has a medicine bottle with a memory unit attached thereto. However, the memory unit allows only for information to be transferred from the memory unit to a playback unit. No information is written from the playback unit to the memory unit. Therefore, no information with regard to actual use of the medication is carried in the memory unit. Other devices are used to indicate when the product has passed its expiration date. This can be very important with regard to medicines because after that date they may not have any value or they may become toxic. An example of such a device is US patent 5,802,015 issued September 1, 1998 to Rothschild et al. which shows an electronic label that indicates the expiration of a predetermined time period.

None of the prior art systems described above show a package with an interactive memory unit that both stores information with regard to the medication and the use of the medication by an individual. The package would be used in association with a consumer prompting device that both reads information from the package and writes information onto the package with regard to use of the medication. The information with regard to the use of the package is stored on the interactive memory unit, that is the parameters for determining the next take time. The parameters for determining the next take time are transferred to the consumer prompting device wherein the next take time is calculated and the next take time is stored on the consumer prompting device and the parameters for determining next take time is deleted from the consumer prompting device. In addition, if the consumer takes a medication the take time is written from the consumer prompting device to the interactive memory unit. Thus, all information with regard to the medication is stored with the package. The advantage of the system herein is that the consumer prompting device does not store each set of parameters for determining the next take time for each medication, rather it obtains the parameters from the package, calculates the next take time and then deletes the parameters. Accordingly, the memory requirements for the consumer prompting device can be greatly reduced. In addition information with regard to use of a medication could also be stored with the consumer prompting device. This information can be stored in full or with codes corresponding to specific information wherein the memory device has limited memory capacity.

Accordingly, it would be advantageous to provide a package that includes a memory unit that stores information with regard to the medication and in addition stores information with regard to the use thereof. Thus the package could electronically store information with regard to the medication and its incompatibilities, the particular regime for a particular individual, and information with regard to corrective measures when the medication is not taken in accordance with the preferred instructions. When such a package is used with a consumer prompting device, the device can prompt the individual with regard to the regime and the consumer prompting device can write back onto the memory unit in the package information with regard to use of the medication. The consumer prompting device can be arranged such that the prompt for taking the medication can only be deactivated by the particular package. Further, the consumer prompting device may prompt for refills, provide a second log of the use of a particular medication, and provide a positive identification of the individual with the package. The consumer prompting device may include a display that advises regarding corrective measures where the prescribed regime was deviated from.

The amount and nature of the information stored on the memory unit attached to the package will depend on the capacity of the memory unit. Where there is limited memory the information will be stored using alphanumeric codes that will correspond with standard information stored on the consumer prompter. For example take on a full stomach/ take with a glass of water/do not take before strenuous exercise and the like, the full text of which is stored on the consumer prompter and corresponds to alphanumeric codes stored on the interactive memory unit attached to the package.

A package having a memory unit when used in association with a consumer prompting device would provide a number of advantages. Information with regard to the use of the medication would be attached to the package of medication. This would help the health care professional to diagnose the individual when considering symptoms and further medications. Further, such information would be useful for clinical studies with regard to the use of medication. Further, the package in association with the consumer prompting device provides a system of verification to help reduce the chance of an individual taking a medication at the wrong time or in association with incompatible medications.

In regard to on-line sales of pharmaceuticals it would be advantageous to provide a method for verifying the authenticity of prescriptions submitted via the internet to on-line pharmaceutical sales web sites (i.e. verify that the prescription was prepared by an authorized medical doctor following required procedures). Further it would be advantageous if such a method could effectively and easily monitor compliance to on-line sales regulations and track the process from authentication to delivery. Still further it would be advantageous if such a method could be easily integrated into a traditional retail pharmacy dispensing operation and easily integrated with a consumer prompting device and a package having an integrated circuit chip attached thereto.

Further, it would be advantageous to provide a method of checking for medication conflicts at the point of purchase. Further it would be advantageous for such a method to be adaptable for use by an on-line pharmacy and/or a retail pharmacy. Such a method would enable the pharmaceutical companies as well as the government to provide further safety measures to reduce the chances of a patient taking incompatible drugs.

## SUMMARY OF THE INVENTION

With the present invention, it is assumed that patients will have an interactive reminder device and that this can reliably be identified by the patient and not get mixed up with a similar device belonging to another patient. In the home and hospital contexts, this is entirely reasonable, particularly where the device also serves as another kind of device that is personal to an individual, such as a personal organiser/PDA or mobile telephone. The present invention then proposes that a unique number, stored on the personal interactive reminder device, is written to a smart label on a pill bottle when it is first used. This reliably links the bottle to that particular personal interactive reminder device. Subsequently, the device prompts the patient to take medication at the correct time: the patient brings the device in proximity to the smart label on the pill bottle so that the unique identifier stored on the smart label can be compared to the identifier stored on the personal interactive reminder device: only if there is a match is the patient prompted to take the medication from that bottle by the device. Hence, the critical insight of the present invention is writing a unique identifier to each smart label in order to positively associate that label with a specific reminder device and hence a specific individual. Further details and aspects of the invention are defined in the claims.

The prior art teaches no such automatic and reliable linkage between a reminder device and medication: for example, some prior art systems either ignore the need for this kind of linkage (eg. US 4695954) or assume that it can be done manually (eg. US 5852590) by a patient reading the name of the patient displayed on the reminder device to check that the medication is indeed theirs. This is not as reliable as the present invention.

## BRIEF DESCRIPTION OF THE DRAWINGS

The invention will now be described by way of example only, with reference to the accompanying drawings, in which:

Figure 1 is a front view of a blister pack for pills having an integrated circuit chip embedded therein in accordance with the present invention;
Figure 2 is a perspective view of a pill container having an integrated circuit chip embedded therein in accordance with the present invention;
Figure 3 is a front view of a tube of medication having an integrated circuit chip embedded therein in accordance with the present invention;
Figure 4 is a top perspective view of a consumer prompting device in accordance with the present invention;
Figure 5 is a bottom perspective view of the consumer prompting device of figure 4 as viewed from arrow 5;
Figure 6 is an end view of the consumer prompting device of figure 5 as viewed from arrow 6;
Figure 7 is a bottom view of the consumer prompting device of figure 4;

Figure 8 an enlarged perspective view of a pill container;
Figure 9 is an bottom view of the pill container of figure 8;
Figure 10 is a bottom view showing the pill container of figure 8 in contact with the consumer prompting device of figure 4;
Figure 11 is a cross sectional view of the pill container in contact with the consumer prompting device taken along line 11 of figure 10;
Figure 12 is a bottom view of a blister pack of figure 1 moving into contact with the consumer prompting device of figure 4;
Figure 13 is a bottom view of a tube of figure 3 moving into contact with the consumer prompting device of figure 4;
Figure 14 is a perspective view of an alternate embodiment of the consumer prompting device using a contactless system;
Figure 15 is a perspective view of a pill container having an integrated circuit chip attached thereto with a label with a portion of the label shown broken away;
Figure 16 is a top perspective view of a device used by a health care professional for inputting information into an integrated circuit chip attached to a package in accordance with the present invention;
Figure 17 is a bottom perspective view of a device used by a health care professional shown in figure 16;
Figure 18 is a block diagram of the elements inside a consumer prompting device;
Figure 19 is a flow chart showing the steps used to program an integrated circuit chip attached to a package;
Figure 20 is a flow chart showing the steps used by an individual;
Figure 21 is a flow chart showing the logic steps of the consumer prompting device;
Figure 22 is a flow chart showing the use of the present invention in a health care facility;
Figure 23 is a flow chart showing the steps to register a doctor on an authentication web site;
Figure 24 is a flow chart showing the steps to register a pharmacy on an authentication web site;
Figure 25 is a flow chart showing the steps for an online purchase of a pharmaceutical;
Figure 26 is a flow chart showing the steps of a portal site interaction; and
Figure 27 is a flow chart showing the steps to detect medication conflicts.

## DETAILED DESCRIPTION OF THE INVENTION

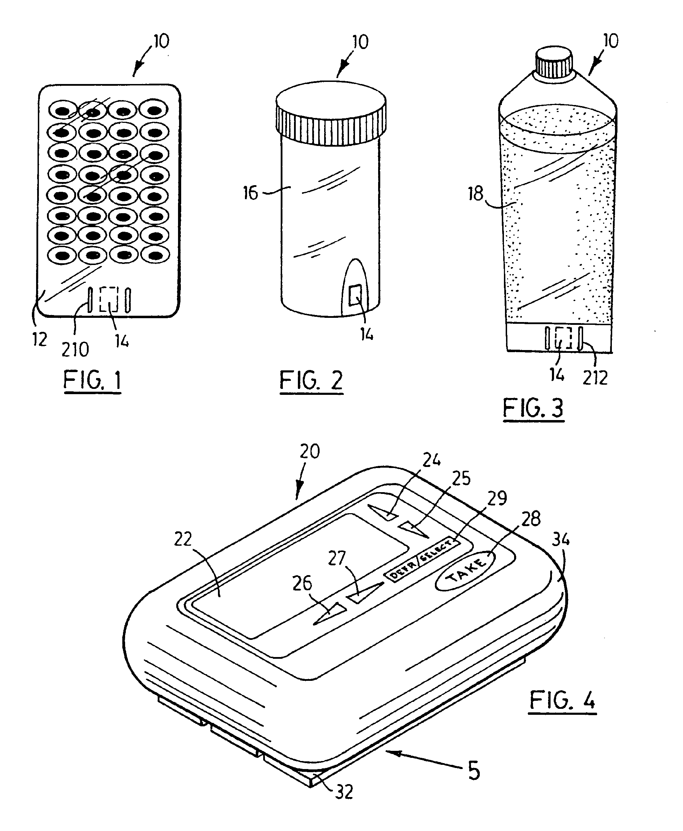

Referring to figures 1 through 3, a package with an integrated circuit chip attached thereto is shown generally at 10. Three different examples of packages are shown in figures 1 through 3. Specifically, figure 1 shows a blister pack 12 with an integrated circuit chip 14 attached thereto, figure 2 shows a bottle 16 with an integrated circuit chip 14 attached thereto and figure 3 shows a tube 18 with an integrated circuit chip 14 attached thereto. Preferably, integrated circuit chip 14 is embedded in the package 10 such that removing the integrated circuit chip 14 results in damaging the package.

The integrated circuit chip 14 is used to store information about the product in the package and about the use of the package. This is of particular importance in regard to the medications. Specifically, parameters for determining the next take time are stored on the integrated circuit chip 14. As well information regarding the medication such as best practices regarding administering the medication, incompatible medications, amount of medication in the package, and corrective action if the medication is not taken in accordance with the best practices may be stored on the integrated circuit chip 14. In addition the integrated circuit chip can include information specific to the patient such as patient identification and the specific regime for the patient. The integrated circuit chip can also store information with regard to the dates and times the medication was used.

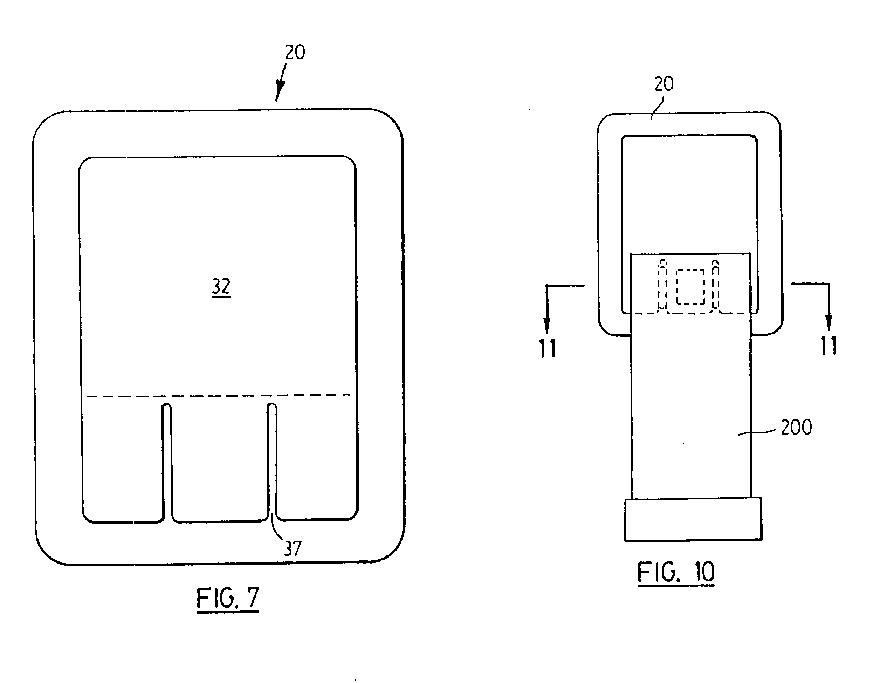

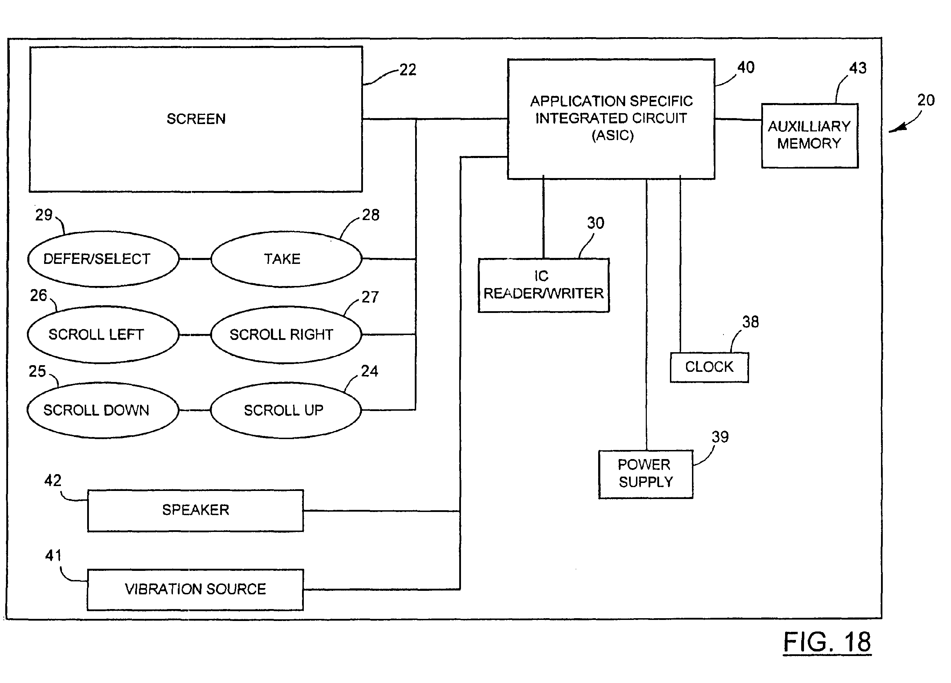

Referring to figures 4 to 7, consumer prompting device is shown generally at 20. Consumer prompting device 20 includes a display 22, a scroll up arrow shaped button 24, a scroll down arrow shaped button 25, a scroll left arrow shaped button 26 and a scroll right arrow shaped button 27. The scroll buttons 24, 25 allow the user to scroll the information in the display screen up or down respectively. Scroll buttons 26 and 27 allow the user to scroll the information in the display screen left and right respectively. In addition there is a TAKE button 28 and a DEFER/SELECT button 29. Consumer prompting device 20 has a read/write module 30, shown in figure 18. Read/write module 30 is a contact type read/write module wherein contact is required. Consumer prompting device 20 can be any size or shape desired. Preferably consumer prompting device is the size of a pager.

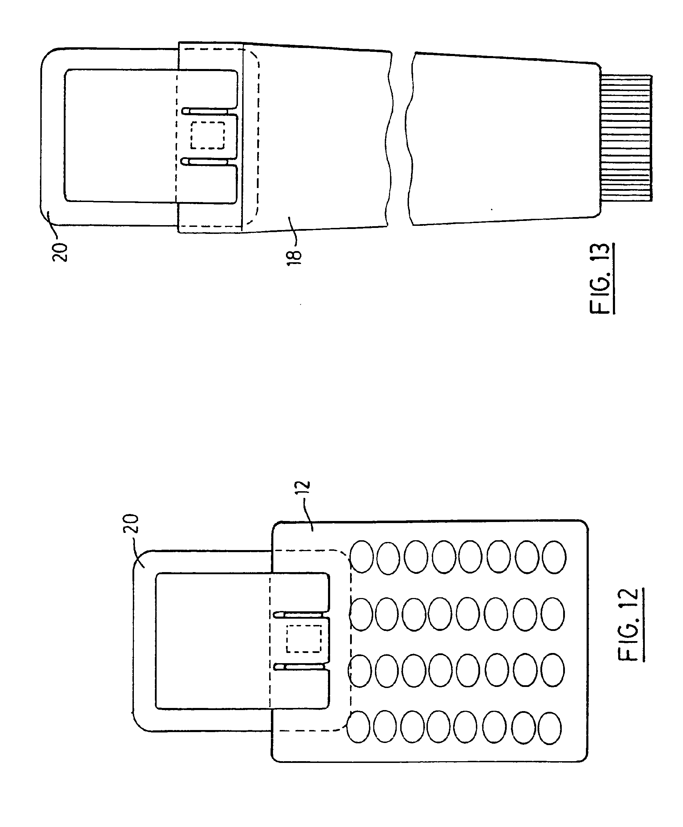

A read/write station 32 is attached to case or housing 34. Read/write station 32 includes a longitudinal slot 36 and a pair of transverse slots 37. The longitudinal slot 36 facilitates bringing the integrated circuit chip 14, in blister pack type packages 12 or tube 18 as shown in figures 12 and 13 respectively, into contact with the read/write module 30 housed in the consumer prompting device 20. Since longitudinal slot 36 is open at each end there is no restriction in the width of blister type packages 12 or tubes 18 that can be used. Transverse slots 37 are used to position the integrated circuit chip 14 in the read/write station 32 such that integrated circuit chip 14 is in contact with read/write module 30 in the consumer prompting device 20.

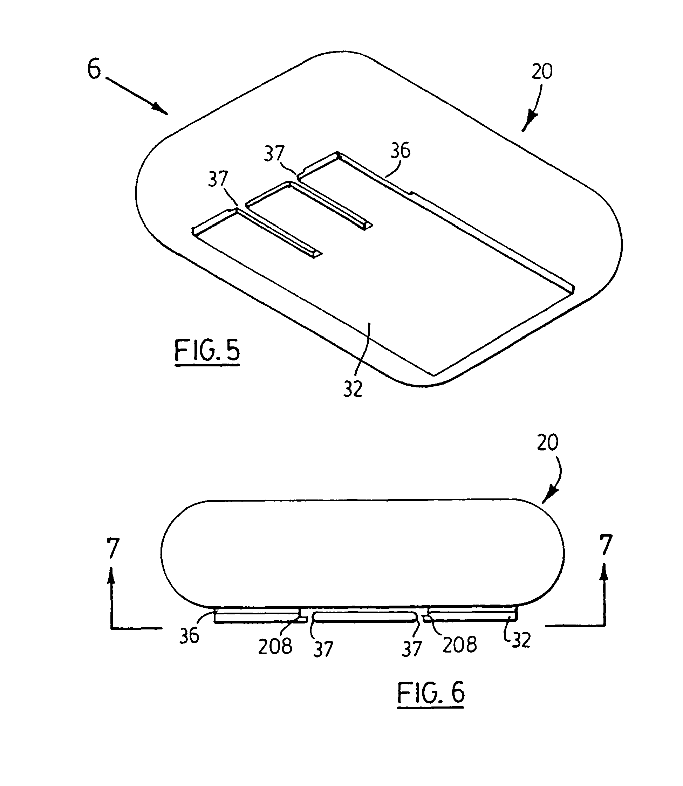

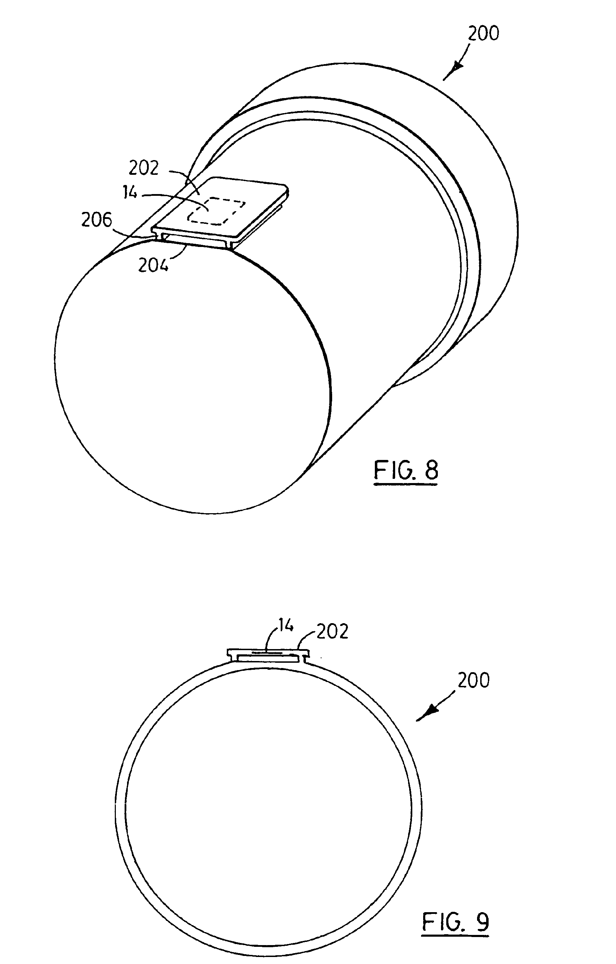

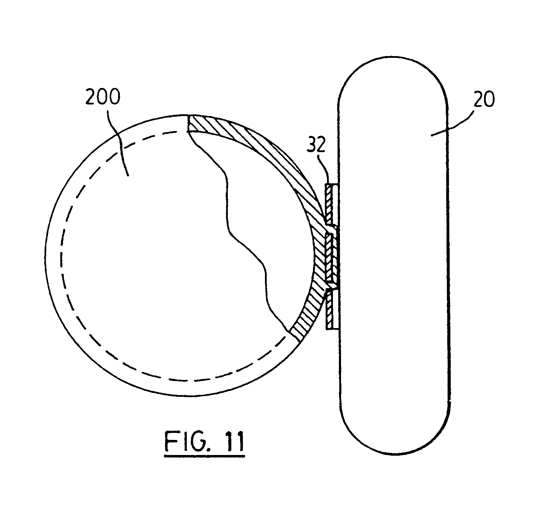

Referring to figures 8 to 10 a bottle 200 includes an integrated circuit chip 14 that is attached to a plate 202 that is spaced from bottle wall 204. Legs 206 are attached to bottle wall 204 and plate 202 and hold plate in position. Since the plate 202 is spaced from the wall 204 bottle chip 14 can come into contact with reader/writer module 30 as shown in figures 10 and 11. Legs 206 slide into transverse slots 37. Transverse slots 37 are provided with ledges 208 (best seen in figure 6) to further aid in the positioning of the bottle 200 in reader/writer station 32.

The integrated circuit chip 14 in blister type package 12 is positioned in the read/write station 32 as shown in figure 12 so that chip 14 is in contact with reader/writer module. Blister type package 12 includes a pair of spaced apart ridges 210 that are positioned on either side of integrated circuit chip 14 and are positioned to be in registration with transverse slots 37 when the blister type package 12 is positioned in the reader/writer station 32. Similarly, the integrated circuit chip 14 in tube 18 is positioned in read/write station 32 as shown in figure 13 so that chip 14 is brought into contact with reader/writer module. Blister type package 12 includes a pair of spaced apart ridges 210 that are positioned on either side of integrated circuit chip 14 and are positioned to be in registration with transverse slots 37 when the blister type package 12 is positioned in the reader/writer station 32. Similarly, tube 18 includes a pair of spaced apart ridges 212 that are positioned on either side of integrated circuit chip 14 and are positioned to be in registration with transverse slots 37 when the tube 18 is positioned in the reader/writer station 32.

Referring to figure 18, consumer prompting device 20 includes a clock 38, a reader/writer module 30, a screen 22, a power supply 39, an integrated circuit 40, scroll buttons 24, 25, 26, and 27, TAKE button 28, DEFER/SELECT button 29, vibration source 41 and speaker 42. In addition there may be auxiliary memory 43. Preferably the screen 22 is a liquid crystal screen. The integrated circuit 40 is an application specific integrated circuit. Preferably the power supply 39 is an AA battery. Alternatively or in addition, the consumer prompting device could include a light indicator.

A menu screen with the aid of the scroll buttons 24, 25, 26 and 27 will make available all of the information stored in the consumer prompting device 20. Specifically information such the medication list and the next take times, the date and time set, time zone adjustments, volume control for the audio prompt, selecting prompt method, and delete and stop medication may be viewed. Preferably the user can select the prompt from a menu including vibration, various audio prompts and a visual indicator on the screen. The time zone adjustment allows the user to set his then current time zone and when the user changes time zones and a new time zone is entered the next take times will be adjusted for the new time zone. When the integrated circuit 14 is read by the read/write module additional information is available through the menus on the screen, namely best practices, contra indicators and hazards. In addition the frequency and dosage for the medication, the prescribing physician and any other information stored on the integrated circuit chip 14 are available.

Device 20 would record the number of times that the date and time set was adjusted. This would allow the health care professional to determine if the record of take times is accurate and to ensure that the user cannot circumvent the monitoring aspect of the present system by changing the date and time set to falsely record the date and time a medication was taken.

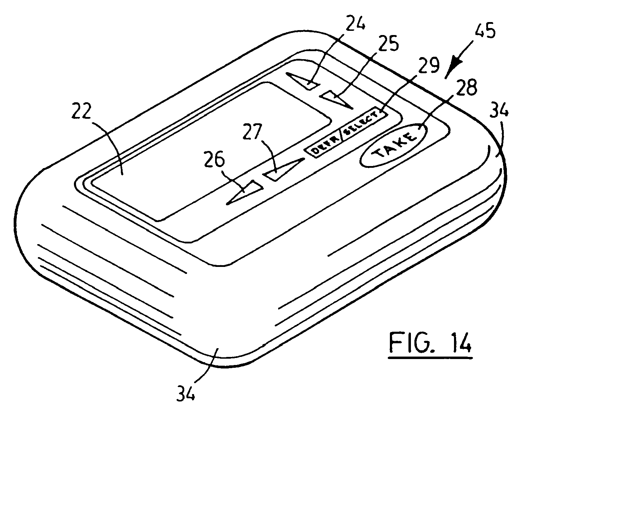

Referring to figure 14 alternatively, a contactless read/write module is used in the consumer prompting device 45. The main difference between consumer prompting device 45 and device 20 is that device 45 includes a contactless read/write module. Accordingly consumer prompting device 45 does not require a read/write station *per se*, since the integrated circuit chip 14 need not actually contact the contactless read/write module. The contactless read/write module is positioned proximate to the surface of the device 45. To facilitate use of a contactless read/write module an antenna is provided in the package attached to the integrated circuit chip 14. In use the integrated circuit chip 14 is brought into proximity with the contactless read/write module and information is transferred between the consumer prompting device and the integrated circuit chip. Thus the consumer can "swipe" the package by device 45 to exchange information. Preferably consumer prompting device 45 will indicate when information has been transferred. The indicator could be a visual indicator, a sound or the like.

It will be appreciated by those skilled in the art that the consumer prompting device 20, 45 could be incorporated into a Palm Pilot™, an electronic personal organiser, a cell phone, a personal digital assistant, hand held computer or another electronic device that is easily carried by an individual. In particular the read/writer module and a read/write station where required could be incorporated into such a unit. The functionality of the consumer prompting device 20, 45 could be incorporated into any device that includes a read/writer module and where required a read/write station. However, preferably it would be incorporated into a device that the consumer routinely always carries. Thus the consumer would not need to carry a separate device.

Further, it will be appreciated by those skilled in the art that the consumer prompting device 20, 45 could use a touch screen rather than the specific buttons shown herein. Any or all of the specific buttons could be replaced by electronic buttons on a touch screen.

Referring to figure 2, a bottle 16 is shown with an integrated circuit chip 14 embedded therein. This type of bottle 16 is for use with a contactless consumer prompting device 45. With regard to blister type package 12 and tube 18 ridges 210 and 212 respectively need not be included when used in association with a contactless prompting device.

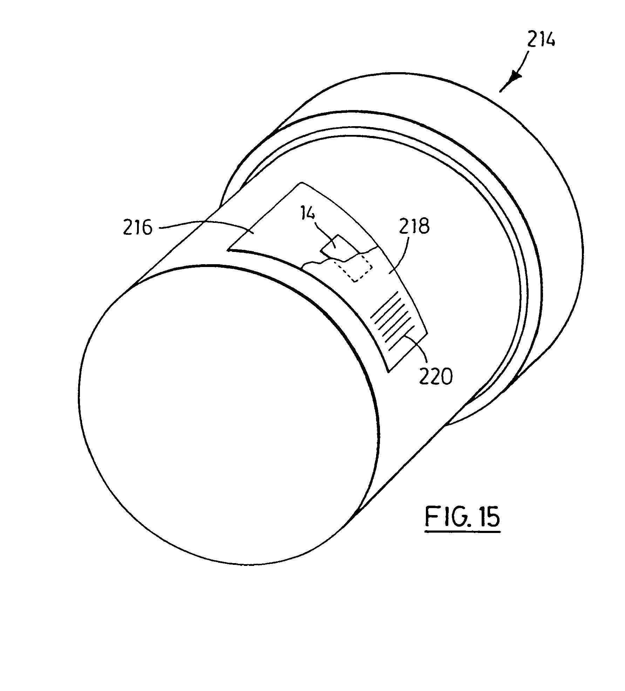

Referring to figure 15, a bottle 214 is shown with the integrated circuit chip 14 attached thereto with a label. The label includes an adhesive layer 216, the integrated circuit chip 14 and a top layer 218. Top layer may include any information desired by the user including a bar code 220. A label with an integrated circuit chip 14 therein will be referred to herein as a smart label and it will be appreciated by those skilled in the art that any package could have a smart label attached thereto and all of the information stored on integrated circuit chip 14 embedded in a package 10 could also be stored on an integrated circuit chip 14 attached to a package by a smart label. Hereinafter all references to integrated circuit chip 14 could mean a chip embedded in a package 10 or a chip in a smart label. Where the term sticky label is used this is separate from a smart label. A sticky label is used to designate the kind of labels currently found on packages.

It will be appreciated by those skilled in the art that the storage capacity of an integrated circuit chip can vary widely. The above description assumes a storage capacity in the order of 256K. Alternatively a chip with considerably less capacity could also be used. Where there is limited storage capacity, certain pieces of information will be stored as an alphanumeric code that is readable or interpretable by the consumer prompting device 20, 45. In addition certain pieces of information may be eliminated. For example, since information regarding the doctor and the patient will be found on the sticky label this need not be encoded onto the integrated circuit chip. Either type of integrated circuit chip will include the parameters for determining the next take time, the unique identifier from the consumer prompter, the issue date, the prescription number created by the pharmacy and the medication name. In addition it will include the dosage, the number of pills, the repeats allowed and the number of pills remaining. Where there is limited storage capacity the medication name or trade name will be digitally encoded. Further where there is limited storage the warnings, contra-indicators and corrective measures will be encoded to correspond to list of specific instructions.

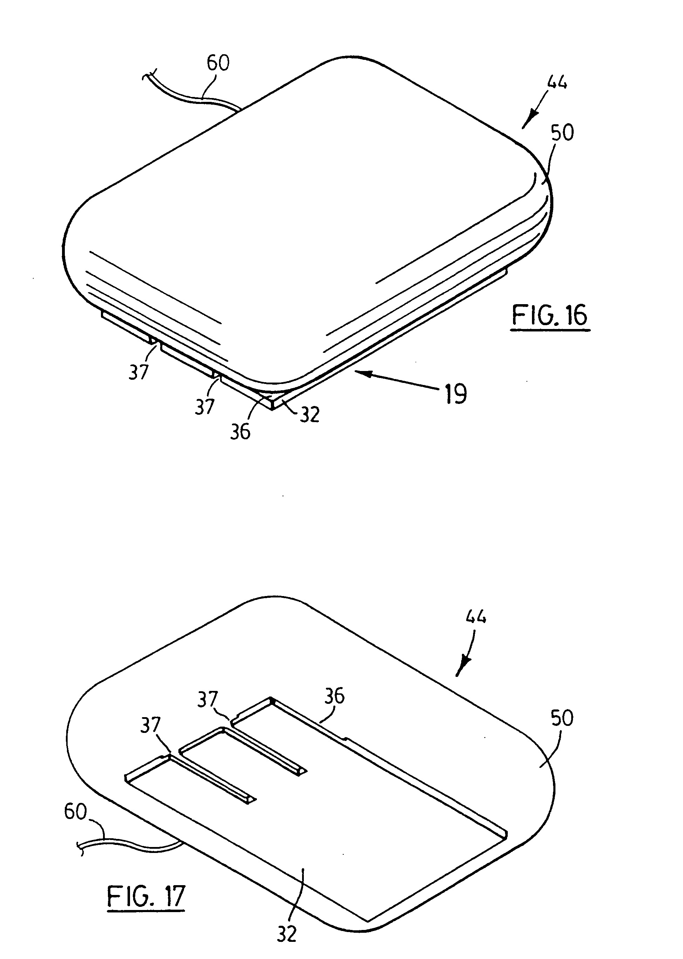

Referring to figures 16 and 17 a reader/writer 44 is for use by a health care professional such as a retail pharmacist, a hospital pharmacist, a doctor, a doctor's assistant and the like. Reader/writer 44 is somewhat similar to the consumer prompting device 20 discussed above. Reader/writer 44 has a chip read/write module. Similar to consumer prompting device, read/write module is a contact type read/write module wherein contact is required. Accordingly, a read/write station 32 as described above is provided in the case 50 of reader/writer 44 to facilitate bringing the integrated circuit chip 14 in blister pack type packages 12 or tube 18 or bottle 16 in contact with the read/write module. The reader/writer 44 is connected through cable 60 to the health care professional's computer (not shown) for inputting information into the integrated circuit chip 14 in the package. Information specific to the patient or consumer is written onto the integrated circuit chip 14. In addition information such as contra indicators, warnings, cautions or procedures to take if a patient fails to take the medication at the prescribed time may be written onto the integrated circuit chip by the health care professional or by the pharmaceutical company if it is an integrated circuit chip that is always associated with a specific medication. It will be appreciated by those skilled in the art that alternatively if desired a single purpose reader/writer unit could be developed for inputting the information onto the integrated circuit chip rather than connecting the reader/writer 44 to the health care professional's computer.

A package having an integrated circuit chip 14 attached thereto could be used in monitoring the flow of packages in a number of different applications. The application described hereafter is for use with regard to monitoring and prompting for the correct use of medications. In order to facilitate the correct use of the medications a number of systems need to be implemented. Specifically there is a system for obtaining the information specific to a patient or consumer and writing it on an integrated circuit chip attached to the medication; a system for ensuring that the patient takes the right medication at the prescribed time; a system used by the consumer prompting device to prompt the patient or consumer; and a system that modifies the above systems for use in a health care facility.

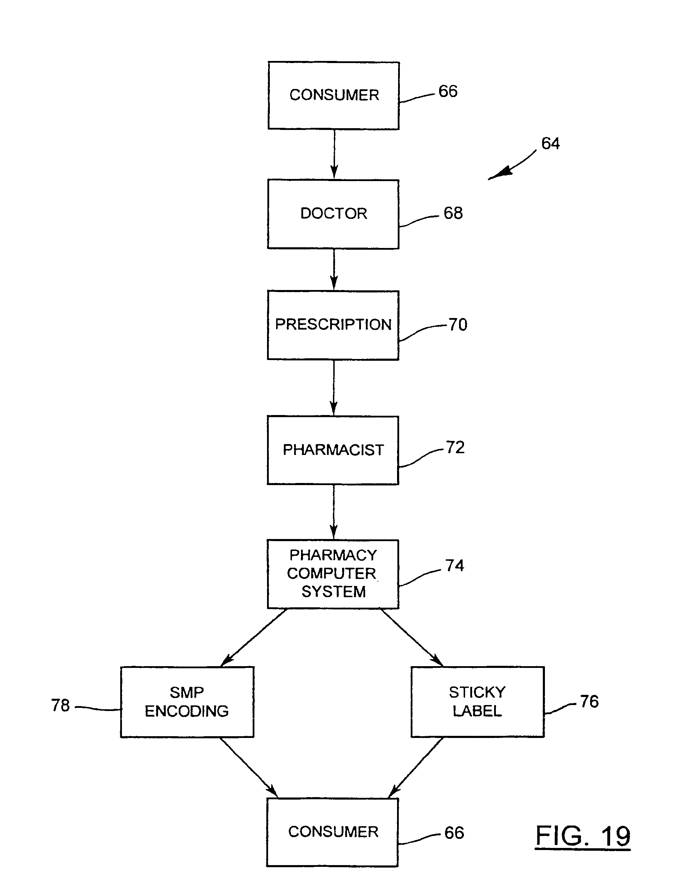

Referring to figure 19 the steps for obtaining the information specific to a patient or consumer and writing it on an integrated circuit chip attached to the medication is shown generally at 64. The patient or consumer 66 is attended to by a doctor 68. The doctor provides a prescription 70. The prescription may be in the form of the traditional written script or electronic information that is provided on a smartcard or the like. The patient 66 attends at a pharmacy and the pharmacist 72 inputs information with regard to the prescription 70 in the pharmacy computer system 74. The pharmacy computer system 74 generates a sticky label 76 and encodes 78 information onto the integrated circuit chip 14. Information such as prescribed manner of taking medication including time and dosage is encoded or written onto the integrated circuit chip, as well parameters for determining the next take time are encoded or written onto the integrated circuit chip. In addition, information with regard to best practices, contra indicators and hazards is written onto integrated circuit chip 14. The medication with the sticky label 76 and integrated circuit chip 14 attached thereto is given to the consumer or patient 66.

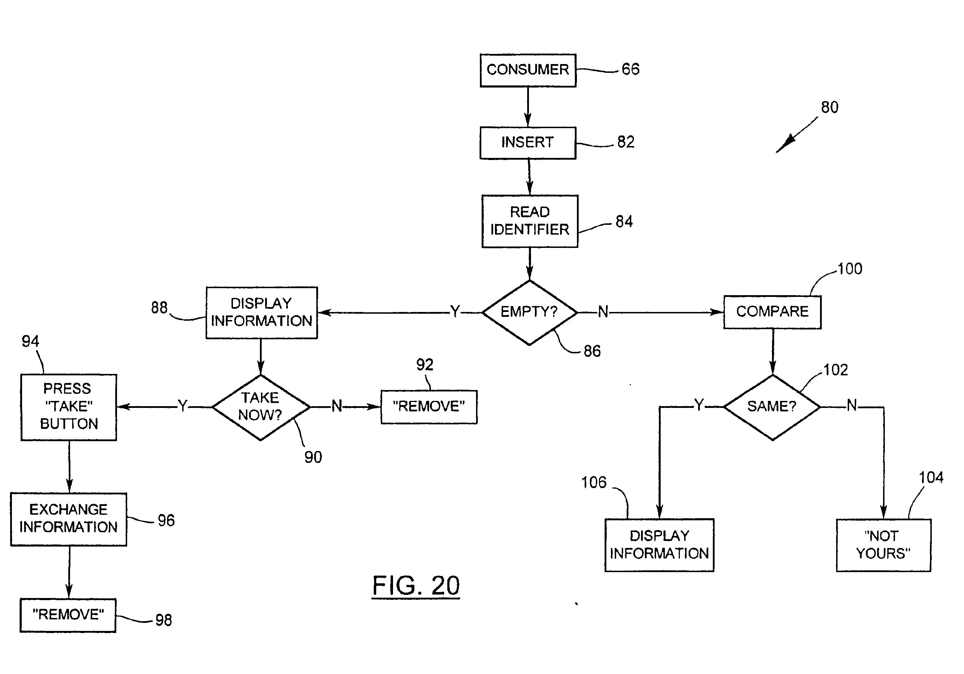

Information such as the contra indicators and hazards may be written onto the integrated circuit chip 14 by the pharmaceutical company prior to shipping. This provides the pharmaceutical company with the opportunity of updating the information with regard to best practices, contra indicators and hazards as soon as the information is available and prior to shipping. The parameters for determining the next take time could include a wide range of variables. Preferably the next take time includes a take window wherein the next take time is the preferred take time but there is a time range where taking the medication is acceptable. This would be useful for a user who wanted to take a medication prior to starting an activity, the user could insert the medication package 10 into the consumer prompting device 20 and determine if the time is within the take window. Preferably the next take time also includes defer parameters. For example in the case of certain conditions wherein failure to take the medication at the prescribed time is life threatening if user defers taking the medication the device will prompt again in a very short period of time. Alternatively where failure to take the medication at the prescribed time is not life threatening the prompt may be deferred for a longer period of time. In addition, preferably information with regard to the number of medications in the package will be included in the information stored on the integrated circuit chip14, the date and time of taking a medication and the amount of medication left will be updated each time a medication is taken. Thus when a predetermined amount of medication is left information with regard to a obtaining a refill will be transferred to the prompter which will prompt the consumer to buy a refill. The steps that the patient takes in association with a package having an integrated circuit chip 14 and the consumer prompting device 20 for ensuring the right medication at the prescribed time is shown generally at 80 in figure 20. The consumer or patient 66 inserts 82 the package or medication with the integrated circuit chip 14 into the consumer prompting device 20. The consumer prompting device reads the unique identifier 84 on the integrated circuit chip 14 attached to the medication. Device 20 determines if the field is empty 86. If the field is empty it is a new package and the information in the integrated circuit chip 14 is then read and displayed 88, including such information as best practices with regard to taking the medication, contra indicators and hazards. In addition, if the field is empty the name of the consumer is displayed and if the name is correct the consumer can press the TAKE button or the DEFER button and a unique identifier is written onto the integrated circuit chip 14 of the package 10. Where the consumer has pressed the TAKE button, device 20 read the parameters for determining the next take time and determined that the medication should or could be taken at that time 90. Where the consumer pressed the DEFER button, device 20 read the parameters for determining the next take time and determined that the medication should not be taken at that time or could be deferred. If the consumer pressed DEFER, the package is removed 92 from the consumer prompting device. If the consumer pressed TAKE 94, information regarding the taking of the medication is exchanged 96 between the consumer prompting device 20 and the integrated circuit chip 14. The parameters for determining the next take time is transferred temporarily from the integrated circuit chip 14 to device 20 wherein the next take time is calculated and stored. Thereafter the parameters for determining the next take time are deleted from device 20. Thereafter the consumer is prompted 98 to remove the package from device 20.

On the other hand if the unique identifier field is not empty, device 20 compares 100 the unique identifier with identifier stored on the consumer prompting device 20. If device 20 does not have the same identifier 102, then the package with the integrated circuit chip 14 is not for use in association with that particular consumer prompting device 20 and it will display 104 "NOT YOURS" and prompt for the package to be removed from device 20. Alternatively, if the identifiers are the same 102 it is a package that is to be used in association with that particular consumer prompting device 20 and information stored on the integrated circuit chip 14 is displayed 106. Preferably the information displayed includes the drug name, the next take time and if the time is currently within the take window, best practices, contra indicators, hazards, prescribing physician and the like. If the device 20 has prompted the consumer to take a medication, the device will confirm that the unique identifier is correct and the device will determine if the medication is the correct medication. Alternatively if the device has not prompted the consumer to take a medication but the consumer would like to take a particular medication at that time, say before the consumer goes out, the device will not only confirm that the unique identifier is correct but also if the time is within the take window for that medication. Accordingly, device 20 reads the parameters for determining the next take time and determines if the medication could be taken now 90. If no, the package is removed 92 from the consumer prompting device. If yes and the consumer intends to take the medication now, the TAKE button is pressed 94, information regarding the taking of the medication is exchanged 96 between the consumer prompting device 20 and the integrated circuit chip 14. Parameters for determining the next take time are transferred temporarily from the integrated circuit chip 14 to device 20 wherein the next take time is calculated and stored. Thereafter the parameters for determining the next take time are deleted from device 20. Information regarding the date and time of the patient taking the medication is transferred from the device 20 to the integrated circuit chip 14. Thereafter the consumer is prompted 98 to remove the package from device 20.

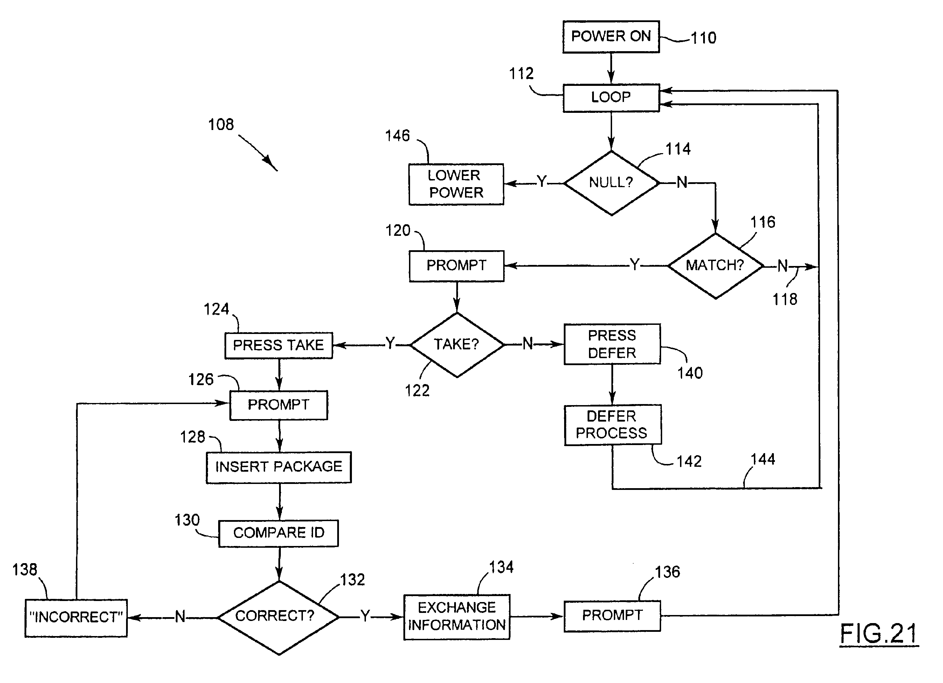

Referring to figure 21 the logic steps of the consumer prompting device 20 to prompt the patient or consumer is shown generally at 108. For the consumer prompting device to be in the active mode the power is on 110. Device 20 loops through the "next take" fields 112. Device 20 determines if the "next take" fields are null 114. If the "next take" fields are not null, that is there is information in the "next take" fields, device 20 determines if the date and time in the "next take" field is the same as the current date and time 116. If no, device 20 goes back 118 to looping through the "next take" fields 112. On the other hand, if the date and time in the "next take" field is the same as the current date and time, then device 20 prompts to take the medication and displays contra indications 120. The prompt may be an audio prompt, a vibration or a visual prompt or a combination thereof. The patient then decides 122 either to take the pill immediately or to defer taking the pill. If the patient chooses to take the medication, then the patient presses TAKE 124. Device 20 prompts 126 the consumer to insert 128 integrated circuit chip 14 associated with a specific medication. Device 20 compares the unique identifier in the integrated circuit chip 14 with the identifier associated with the consumer prompter 130. If the identifiers are correct 132, information regarding the date and time is exchanged and stored 134. That is, information regarding the method of determining the next take time is transferred temporarily from the integrated circuit chip 14 to device 20 wherein the next take time is calculated and stored. Thereafter the parameters for determining the next take time are deleted from device 20. Information regarding the date and time of the patient taking the medication is transferred from the device 20 to the integrated circuit chip 14. In addition preferably the number of pills in the package is decremented and therefore after each take the actual number of pills in the package should be reflected on the integrated circuit chip 14. Thereafter, device 20 prompts the patient to remove the package 136 and then goes back to looping through the "next take fields" 112. However, if the identifiers are incorrect, the message INCORRECT PACKAGE is displayed 138 and the device goes back to prompt 126 the consumer to insert an integrated circuit chip associated with the medication. If the patient cannot take the medication at the time of being prompted, the consumer may press the DEFER button 140. Thereafter the defer processing steps are performed 142 which include updating the date and time for the "next take" field. Thereafter the device goes back 144 to the step of looping through the "next take" fields 112. In the event that the information in the "next take" fields is null, that is no medications are scheduled to be taken, device 20 goes to lower power mode 146. The device is powered on when a new package is inserted or other adjustments are made.

It will be appreciated by those skilled in the art that the information regarding the date and time patient took the medication could be stored on the integrated circuit chip 14 as discussed above. However, that information could also be stored on the consumer prompting device as well or on another integrated circuit chip such as smartcard health card that contains all patient's health information.

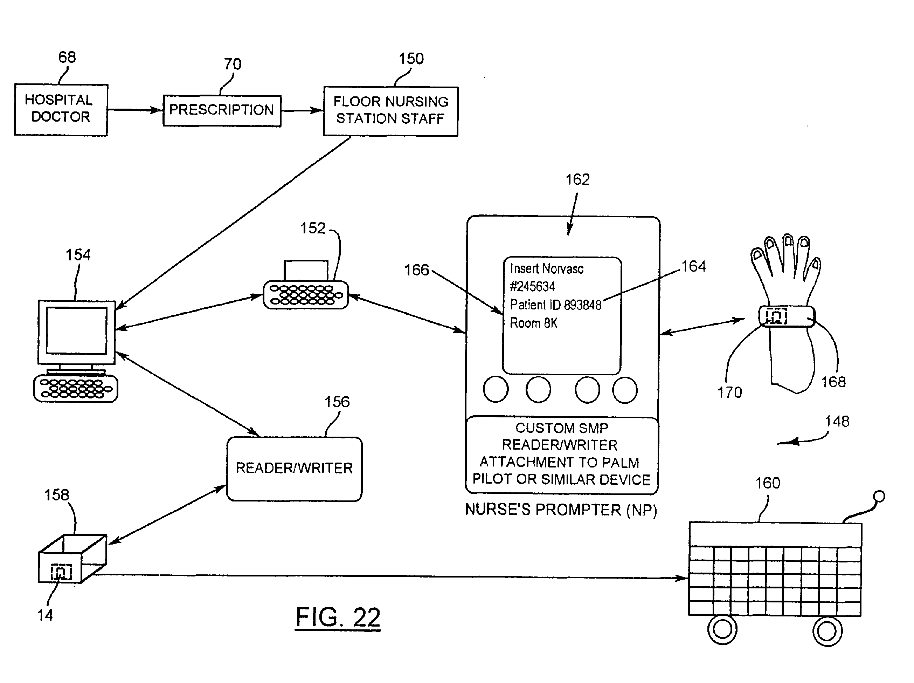

The devices described above can be readily adapted for use in a health care facility such as a hospital. Referring to figure 22, the system for a health care facility is shown at 148. The main components of the hospital system include a hospital medication container 158, independent work station 154 and the nurse prompter 162.

Hospital medication container 158 includes an integrated circuit chip 14. In a typical hospital, medications are dispensed from a central hospital pharmacy, distributed to floor nursing stations or floor pharmacy stations, and then transferred to trays of open containers marked with patient names, floor and room numbers. The hospital medication container 158 includes an integrated circuit chip 14 that is preferably fabricated into a protruding, reinforced 'lip' of the container, which would facilitate the attachment of the container to a hand held reader/writer unit in the nurse prompter 162 and a reader/writer 156 attached to the workstation 154.

Workstation 154 would run nurse prompter host application software, with attached Reader/Writer unit, and keyboard cradle. This independent workstation (Win 95/98/NT, MacOS, or Unix based) would be running the nurse prompter host application software that performs functions supporting the operation of the Nurse prompter (e.g. loading information into Nurse prompter, synchronizing information on Nurse prompter and Nurse prompter-Host, displaying information stored on any Nurse prompter, printing reports, backing up Nurse prompter data, etc.). The attached Reader/Writer 156 is where hospital medication container 158 is attached when interacting with the Nurse prompter-Host application. The keyboard cradle 152 is used to communicate and exchange data with nurse prompters 162. Thus the keyboard cradle 152 can be used to input information into the nurse prompter 162.

Preferably the nurse prompter 162 uses off-the-shelf hardware as the base unit for the nurse prompter (e.g. existing or future versions of personal handheld electronic organizers with sophisticated operating systems, such as the Palm Pilot™, running a Java JVM (Java Virtual Machine) under the Palm OS, or a PSION Series 5MX running a JVM under EPOCH. A reader/writer is added to this base hardware. The reader/writer can be a contact type or a contactless type. The software developed to drive the nurse prompter 162 functionality would use the development tools of the OS of a particular electronic organizer. Interaction with the software would be via the stylus activated touch screens and/or attachable keyboards of the organizers. The nurse prompter 162 could be associated with any group of rooms/beds, and information could be passed between nurse prompters 162.

Referring to figure 22, the hospital system has a number of steps that are common to the individual system described above. The patient would be seen by a doctor 68 and more specifically a hospital doctor who would write a prescription 70. The prescription information would be given to the floor nursing station staff 150 or the staff responsible for administering the medication to the patient 150. The prescription information would also be given to the hospital pharmacy computer system. The information would be given to the floor pharmacy station 154 or the unit responsible for dispensing the medication. The pharmacy station 154 would be connected to a read/writer 156 similar to the reader/writer 44 above. It would encode information onto the integrated circuit chip 14 attached to the hospital medication container 158. A plurality of hospital medication container 158 can be put in a hospital medication cart 160 for easy transportation to the patients. The nurse prompter 162 is somewhat similar to the consumer prompting device 20 but it would display additional information such as a patient identification number 164 and a room number 166. The information could be encoded directly into its integrated circuit chip from the hospital reader/writer 156 or from the information on the hospital smart medication container 158. In use, when the nurse prompter 162 indicates that a medication is due the nurse would need to verify that it is the correct medication by reading the information on the integrated circuit chip 14 attached to the hospital medication container 158 and to verify that it is the correct patient by reading the information on the integrated circuit chip 170 attached to the patient's identification bracelet 168. During this verification process information related to the distribution of the medication would be exchanged between the nurse prompter and the patient integrated circuit chip 170 and between the hospital medication container 14. This system will help to reduce the likelihood of administering a medication to a patient more than once. Further, the doctor or other health care professional will be able to read the information on the patient identification bracelet 168 to monitor the amount and time of medication. The device of the present invention is very user friendly and it is designed so that a user can easily use the device without a lengthy training period. Following sets out the steps in a typical example of the use of the consumer prompting device 20 of the present invention:

- the user installs a battery and sets the date and time and time zone on the user prompting device 20;
- user goes to a doctor and is given prescription for one or more medications;
- user goes to a pharmacy and submits prescription (time is 12:00 noon);
- depending on the packaging style used by the pharmaceutical firm for the prescribed medication, the pharmacist would follow the following steps:

a. PHARMACIST COUNTS OUT PILLS FROM A BULK CONTAINER RECEIVED FROM PHARMACEUTICAL FIRM

• pharmacist enters the prescription into his pharmacy computer system (a database including a record, by user name, of all prescriptions issued to that user);
• usually a prescription from a physician includes: name of medication and dosage/strength, number of pills (or some other measure of quantity) and frequency, number of repeats allowed, name of user, prescribing doctor, date issued;
• pharmacist reads from the consumer prompter 130 the medication that the consumer is currently taking and conducts a conflict analysis and where there are incompatibilities the pharmacist can call the physician for further information;
• pharmacist places empty bottle 16, 200 into pharmacy reader/writer unit 44;
• upon issuing a command, the modified pharmacy computer system software would both print a standard exterior sticky label and write the prescription information onto the integrated circuit chip 14 attached to bottle 16, 200;
• simultaneously, the pharmacy computer system software would write additional information pertaining to the medication onto the integrated circuit chip 14 - this information would originate from the pharmaceutical manufacturer and could be integrated into the pharmacist's computer system software in a variety of ways (including via updates through the internet or via information updates supplied on Smartcards)... this additional information pertaining to the medication would include: warnings, contra-indications, corrective measures in the event of an error in self medication, encoded ranges used by the consumer prompting device 20 to calculate the take windows - the time in which it will issue directions to the user to take a medication, encoded safe prescribing ranges used by the consumer prompting device 20 to cross check prescribing information issued by the doctor;
• pharmacist places the sticky-label on the exterior of bottle 16, 200 and fills the bottle with the correct quantity of pills;
• pharmacist gives bottle 16, 200 to user;

b. PHARMACEUTICAL FIRM PRE-PACKAGES THE MEDICATION IN A **BLISTER PACK 12 OR TUBE 18**

- pharmacist enters the prescription into his pharmacy computer system;
- usually a prescription from a physician includes: name of medication and dosage/strength, number of pills (or some other measure of quantity) and frequency, number of repeats allowed, name of user, prescribing doctor, date issued ;
- pharmacist reads from the consumer prompting device 20 the medication that the consumer is currently taking and conducts a conflict analysis and where there are incompatibilities the pharmacist can call the physician for further information;
- the integrated circuit chip 14 on blister packs 12 or tube 18 of medication would already contain warnings, contra-indications, corrective measures in the event of an error in self medication, encoded ranges used by the consumer prompting device 20 to calculate the take window, encoded safe prescribing ranges used by the consumer prompting device 20 to cross check prescribing information issued by the doctor (all supplied and pre-encoded on the integrated circuit chip 14 fabricated into the blister pack by the pharmaceutical firm)
- pharmacist inserts blister pack 12 or tube 18 into pharmacy reader/writer unit 44;
- upon issuing a command, the modified pharmacy computer system software would both print a standard exterior sticky label and write the doctor-supplied prescription information onto the integrated circuit chip 14 attached to blister pack 12 or tube 18;
- pharmacist places the sticky-label on the exterior of the blister pack 12 or tube 18;
- pharmacist gives blister pack 12 or tube 18 to user;

- user takes bottle home and reads bottle instructions: e.g. take 2 pills 3 times per day on an empty stomach;
- user puts package 10 into consumer prompting device 20;
- consumer prompting device 20 displays appropriate information about the contents of the package 10, including name of medication and dosage/strength, number of pills (or some other measure of quantity) and frequency, number of repeats allowed, name of user, prescribing doctor, date issued, PLUS warnings and contra-indications, in a scrollable window (contra-indications are also automatically checked against any other medications already 'loaded' into the consumer prompting device 20 when packages are inserted);
- warnings indicate factors to be considered before taking the medication: e.g. not to be taken on an empty stomach; do not drive while taking this medication; do not consume alcohol;
- user decides to take medication now, so presses TAKE button;
- consumer prompting device 20 writes identifying information onto the Integrated circuit chip 14 and generates an entry in the 'next take time table' for that medication... the 'next take time' entry is also paired with the encoded range for the take window that was stored on the package 10 (this is used later by the Consumer prompting device 20 when it checks to see if a users choice of a time to take a medication falls within allowed ranges set by the doctor and/or the pharmaceutical manufacturer); Consumer prompting device 20 also writes the 'take time' into the 'take time log' on the integrated circuit chip 14;
- consumer prompting device 20 displays message 'REMOVE PACKAGE AND TAKE 2 PILLS'... user removes package 10 and takes pills (N.B. the user is normally prompted to insert the package 10 AFTER the TAKE button has been depressed, which acts to reinforce the action and validate it or confirm it... only in the 'first use' scenario with each new medication is the package 10 already inserted in the consumer prompting device 20 when the user depresses the TAKE button);
- preferably the number of pills in the package 10 is included on the integrated circuit chip 14 and when the user depresses the TAKE button the number of pills is decremented.
- the encoded ranges used by the consumer prompting device 20 to calculate the take window in which it will issue directions to the user to take a medication also indicate that this is a 'daytime' medication (i.e. assumes 16 awake hours, 8 sleep hours, so it is not necessary for the user to wake up in the middle of the night to take the next dose) so the consumer prompting device 20 computes and sets the 'next take time' to 5:30 p.m;
- at 5:30 p.m. the consumer prompting device 20 beeps or vibrates and displays INSERT (medication name)';
- consumer prompting device 20 could display a color image of the medication to be taken, as a cross check for accuracy (e.g. an image of the pill showing the distinctive shape, color and markings on the pill).
- user is at a restaurant with a client and does not wish to be disturbed, so he depresses the DEFER button;
- consumer prompting device 20 examines the encoded ranges used to calculate take window and computes a DEFER time interval list, displayed as a scrolling list on the Consumer prompting device 20 (e.g. 5, 15, 30 minutes, 1 hour, to maximum as stipulated by pharmaceutical manufacturer);
- user anticipates he will be busy for only another 15 minutes, so he scrolls through the list, stops on 15 minutes, and depresses the SELECT button (N.B. DEFER & SELECT are same button);
- after 15 minutes, Consumer prompting device 20 beeps or vibrates and displays INSERT (medication name)' again;
- user inserts package 10 into consumer prompting device 20;
- consumer prompting device 20 displays appropriate information about the contents of the package 10, including the name of the medication PLUS warnings and contra-indications, in a scrollable window;
- consumer prompting device 20 reads warnings and contra-indications from Package 10 and displays them in a scrollable window (e.g. DO NOT TAKE WITH FOOD);
- user just ate, so he depresses the DEFER button again, and selects a longer time interval;
- consumer prompting device 20 prompts user to remove the package 10;
- after the time interval has elapsed, consumer prompting device 20 again prompts to insert the package 10;
- user could either take the medication or continue to defer (if he defers too many times and goes beyond the calculated 'next take time window', the consumer prompting device 20 prompts to insert the package 10 and displays appropriate warnings and corrective actions;
- the next day the user wakes at 5:30 a.m. and decides to take his medication prior to the consumer prompting device 20 having prompted him, so he inserts the package 10 into the consumer prompting device 20;
- the consumer prompting device 20 computes the take window range for that medication (using the encoded ranges stored with the 'next take time') to see if it is within the acceptable range;
- if it is within the acceptable range, consumer prompting device 20 would display 'OK to TAKE';
- if it is outside the acceptable range, the consumer prompting device 20 displays 'TOO SOON TO TAKE';
- consumer prompting device 20 prompts user to remove the package 10; and
- and so on.

As discussed above, the hospital version of the medication prompting device of the present invention is an adaptation of the consumer prompting device 20 and package 10 configuration, with variations in hardware, software, and operation, to suit the needs of a hospital setting. The general aim is the same (mechanized control of the delivery of medication), but adapted for a hospital environment. In the case of the consumer prompting device system, the specific aim is to provide a means for a consumer to easily and effectively schedule and log his self-medication regimen. In the case of the hospital system, the aim is to provide a means for hospital staff to schedule, log and monitor the administration of medication to patients, and at the same time provide a higher level of safety and security in the distribution process.

It will be appreciated by those skilled in the art that it is possible to construct a version of this hospital system that is highly interactive with a hospital's own internal on-line patient database systems. The database systems in widespread hospital use today are produced by a very large number of software vendors, and are custom fitted to a hospital's needs, and consequently are virtually infinite in their variety. However, the same high level functions would be incorporated into each of these systems to implement the system of the present invention. In each case, custom interfaces would be required. The bulk of this interfacing would be centered around avoiding any duplication of effort. Whenever possible, data required by system of the present invention (e.g. patient admission data) would be captured from a hospital's existing database system and passed to the present system. Relevant data from the present system could also be passed back to the hospital's database system.

It is also possible to construct a version of the present system that is for the most part independent of hospital database systems. The following example will therefore be based on this latter version.

For this example, we assume that a hospital floor is divided into nursing stations that are each responsible for a group of rooms. Any number of nurses may be assigned responsibility for these rooms. The following example shows the use of a nurse prompter 162 in a hospital:

- patient is admitted to hospital and a Smartcard or Java Ring style identification bracelet is attached to the patient's wrist. The ID bracelet 168 has an integrated circuit chip 170 attached thereto. Information such as the patient's name, date of admission and hospital admission number is written onto chip 170. Preferably in addition patient specific medication allergies and/or any other relevant warnings are also written onto chip 170.
- hospital physician(s) prescribe medications and pass these prescriptions to the attending floor nurse, who enters them into the patient's chart or other form of record. Patient medication monitoring in a hospital setting must deal with the complication of passing information between rotating staff. The patient chart, in part, fulfills this obligation.
- enter patient information and medications into the nurse prompter-host application running on a workstation 154 in the nurse's station. Entries would include patient's name, room number, hospital ID number and admission date... entries would also include names of medications and dosage/strength, number of pills (or some other measure of quantity) and frequency, prescribing doctor and date issued, warnings, contra-indications.
- order and prepare the medication for each patient in a group of rooms (floor pharmacy nurse)
- distribute the medication to each patient (floor dispensing nurse)
- floor pharmacy nurse would use the nurse prompter-host at the workstation 154 to obtain a printout or on-line screen listing of the medications to be ordered for that shift. Medications would be ordered and delivered to the floor pharmacy station as normal.
- once the medications have arrived at the floor, they are ready to be loaded into the medication containers 158. Each medication container 158 would have a permanent external marking indicating the room number and bed number (or some other external marking system), in addition to the integrated circuit chip 14 fabricated into the container. The floor pharmacy nurse would select a room number and bed number on the workstation 154. This would display the prescribed medications for the patient, as well as all patient information. The nurse would attach the associated medication container 158 to the Reader/Writer 156 and place medications in the medication container 158. The workstation 154 would write identifying information onto the medication container 158 via the Reader/Writer 156. This process would be continued until all medications for each patient have been loaded (this process is similar to the manual procedures currently practised in hospital floor pharmacy stations). Medication containers 158 would then be loaded onto a trolley 160 for use by the floor dispensing nurse.
- depending on the number of nurses assigned to a group of rooms, the floor dispensing nurse would transfer information via wireless technology to one or more nurse prompters 162. Based on the frequency information loaded into the nurse prompter at the time the prescription is entered, the nurse prompter generates a 'next take table' for each patient in the nurse prompter 162. Each 'next take table' would contain entries for every medication the patient has been prescribed. Each nurse prompter 162 would contain 'next take tables' similar to those in the consumer prompting device 20.
- each floor dispensing nurse would take the nurse prompter 162 associated with her assigned rooms with her on her rounds.
- upon arriving at each bedside, the floor dispensing nurse would attach (or swipe) the nurse prompter 162 to the patient's wrist ID bracelet 168. The nurse prompter 162 would open to the identification information it has stored on it for that patient, and display the medication list for that patient. The nurse prompter 162 would indicate which medications should be started/administered now, and which ones are not within their take window.

- floor dispensing nurse would attach the nurse prompter 162 to the medication container 158 associated with that room & bed. Nurse prompter 162 would verify that it is the correct medication container 158 for that patient by comparing the ID information read from the patient's ID bracelet with the ID information stored on the medication container 158. If it is a match, the dispensing nurse would give the medication to the patient and press the MEDICATION DELIVERED field on the screen of the nurse prompter 162 with a stylus pen. The nurse prompter 162 would log the date and time the medication was administered. This process would be repeated for each medication displayed on the nurse prompter 162.
- floor dispensing nurse would repeat this process with each patient in the group of rooms associated with the nurse prompter 162.
- the nurse prompter 162 would also continuously scan the 'next take table' for each medication of each patient, looking for alerts to be triggered. If a medication has not been administered in time, the Nurse prompter would alert the floor dispensing nurse (vibration, followed by a blinking screen, followed by an audible alarm).
- when an alert is triggered (a match between current date and time), the nurse prompter 162 would display a container number, and the patient's name and medication list, and prompt the nurse to administer the medication. The dispensing nurse could either press DEFER on the Nurse prompter screen, or DELIVER and then proceed to the patient's bedside. The nurse prompter 162 would at this point be waiting for the nurse to confirm that she has the right patient by attaching (or swiping) the nurse prompter to the patient's wrist ID bracelet. If it is the correct patient, the nurse prompter 162 would prompt the nurse to attach the associated medication container 158. If the correct medication container 158 is attached, the nurse prompter would prompt to administer the medication and write log data to the nurse prompter
- the workstation 154 would act as a 'supervisor' of the Nurse prompters:

- if new medications are issued for a patient, the workstation 154 would prompt for the connection of the correct nurse prompter 162 to update its 'next take table' (via wireless technology where available)
- the workstation 154 would periodically prompt for the connection of each nurse prompter 162 so that maintenance tasks could be performed (e.g. backing up data from the nurse prompter).
- nurse prompter 162 could display a color image of the medication to be administered, as a cross check for accuracy (e.g. an image of the pill showing the distinctive shape, color and markings on the pill).
- in the case of liquid medications, single use medication container cups with integrated circuit chip 14 attached thereto could be used.

The traditional method of delivering medication to a pharmacy and thereafter to a consumer may be modified by providing an integrated circuit chip in the packages that contain bulk medication. Thereby the pharmaceutical company can update the information with regard to the medication and disseminate it. The pharmacy can then copy the information from the pharmaceutical company directly from the integrated circuit chip into the pharmacy computer system and transfer it to the integrated circuit chip 14 attached to the dispensing package. This is particularly useful where the pharmaceutical company has identified new contra-indicators or hazards.

When considering a system for providing appropriate checks and balances for purchasing pharmaceutical products from an on-line pharmacy, the purchasing system will include a system for registering and thereafter authenticating doctors. Similarly the purchasing system will include a system for registering and authenticating on-line pharmacies. Preferably the purchasing system will be integrated with an electronic health card, the wave of the future if not the present, wherein the health card includes an integrated circuit chip (hereinafter when a health card or a smart health card is referred to, it is assumed to mean a health card which includes an integrated circuit chip). The health card may be used to store information regarding medication in addition to a whole host of other information.

The on-line purchase system includes an authentication web site wherein physicians and pharmacies register and thereafter can be authenticated. The user will be required to have a reader/writer that is connectable to an internet access enabled device. The consumer prompter 20 could be adapted to be an internet access enabled device. Alternatively the user could use a single purpose device that is attached to the internet access enabled device. Further the system requires an electronically readable prescription, preferably this will be a prescription written on a smart health card.

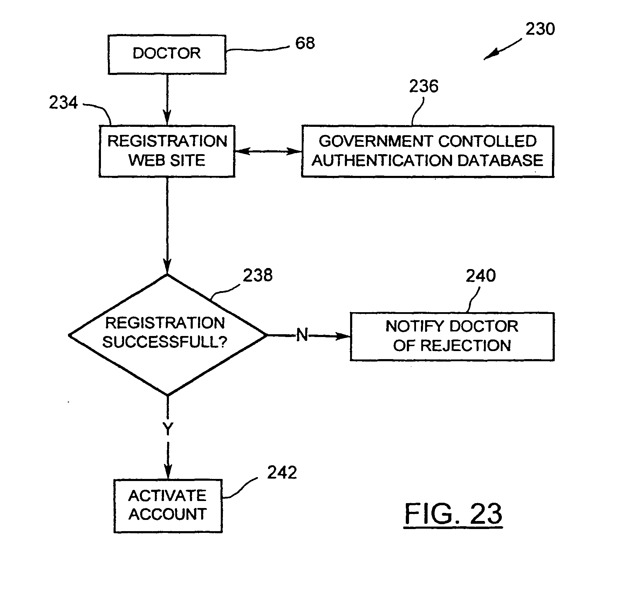

Referring to figure 23 the system for registration of a doctor or physician on an authentication web side is shown generally at 230. Firstly once the doctor or physician 68 chooses to obtain an on-line registration, the physician goes to a registration or authentication web site 234. At the web site 234 the physician submits registration particulars such as name, degree, state license and the like. In addition the physician chooses a password. The physician's information is verified 236 with a government controlled authentication database or other monitored database. Typically the verification is conducted with written and telephone verification. If the authentication process 238 is not successful the physician is notified 240. If the authentication process 238 is successful the physician's account and password is activated 242 and the physician is provided with a physician ID number.

The registration procedure 230 need only be carried out once. Preferably, thereafter certain other security measures are implemented. For example once a month, the physician is e-mailed a new prescription authentication key and prescriptions that are issued by the physician using this key can be filled for a specified time period (e.g. 2 months). Therefore when the physician "writes" a prescription onto a smart health card that prescription will include the physician's ID plus the then current physician's key and together they provide what can be called an e-prescribe key. This relationship can be expressed as follows:

## physician id # + prescription authentication key = e-prescribe key

Preferably the prescription authentication key is delivered by a secure socket layer document delivery web site (similar to Critical Path/DocSpace: www.docspace.com) or other secure method. The physician would enter his password to download the updated prescription authentication key. Preferably the password is changeable at will by the physician in another portion of the web site and optionally the physician is forced to change the password after a specified elapsed time, for example every 6 or 12 months. These are typically the steps that the physician would take on-line and the remainder of the steps would be conducted offline and would be those typically taken by a physician.

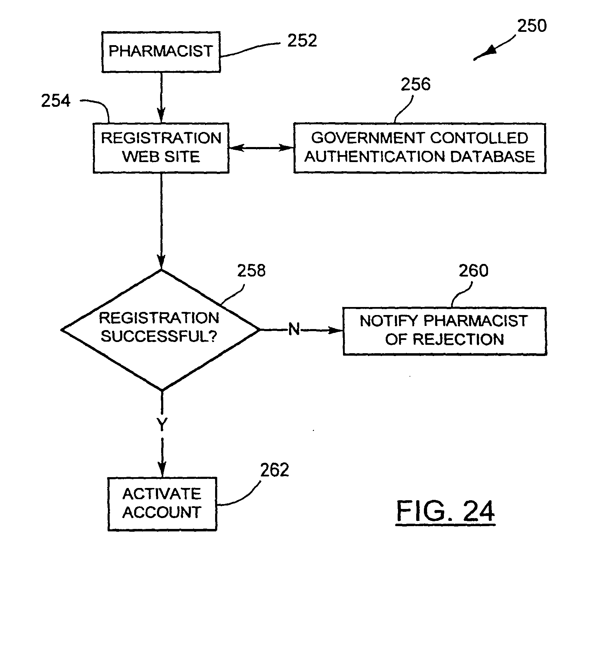

In order to create a secure system for purchasing prescription drugs the on-line pharmacies would need to go through a similar authentication process as that required by doctors. Referring to figure 24 the system for registration of an on-line pharmacy on an authentication web site is shown generally at 250. Firstly once the pharmacist 252 chooses to dispense prescription drugs on-line, the pharmacist goes to a registration or authentication web site 254. At the web site 254 the pharmacist submits registration particulars such as name of pharmacist, the pharmacy site license and the like. In addition the pharmacist chooses a password. The information is verified 256 with a government controlled authentication database or other monitored database. Typically the information is verified using written and telephone communication. If the authentication process 258 is not successful the pharmacist is notified 260. If the authentication process 258 is successful the pharmacist's account and the pharmacist's password is activated 262.

As with the physician, that pharmacist need only register once. Once the pharmacist's account is activated the pharmacist must be provided with (and then periodically updated) the e-prescribe key database which is the combination of the physician ID number and the prescription authentication key. There are a number of ways that this can be updated by encrypted e-mail or other secure method. Alternatively the pharmacist can download the database from a secure web site wherein the pharmacist would use his password. Typically the password can be changed at will by the pharmacist in another area of the web site and preferably the pharmacist has to change his password within a predetermined lapsed amount of time, for example every 6 or 12 months. Preferably the e-prescribe key database is delivered by a secure socket layer document delivery web site (similar to Critical Path/DocSpace) or other secure method.

The e-prescribe key database is consulted when filling a prescription. If the prescription uses a valid e-prescribe key then the pharmacist can fill the order either on-line or at a retail outlet. Alternatively if the e-prescribe key is invalid or missing a key the prescription is considered invalid and the prescription can not be filled or an alternate method of verification can be used. It will be appreciated by those skilled in the art that this method of verifying a prescription can be used in conventional retail pharmacies to enhance their security, it need not be limited to on-line purchases. As discussed above the e-prescribe keys would be valid for a limited predetermined amount of time, for example 2 months, thus allowing the consumer a specified period of time within which to fill the prescription.

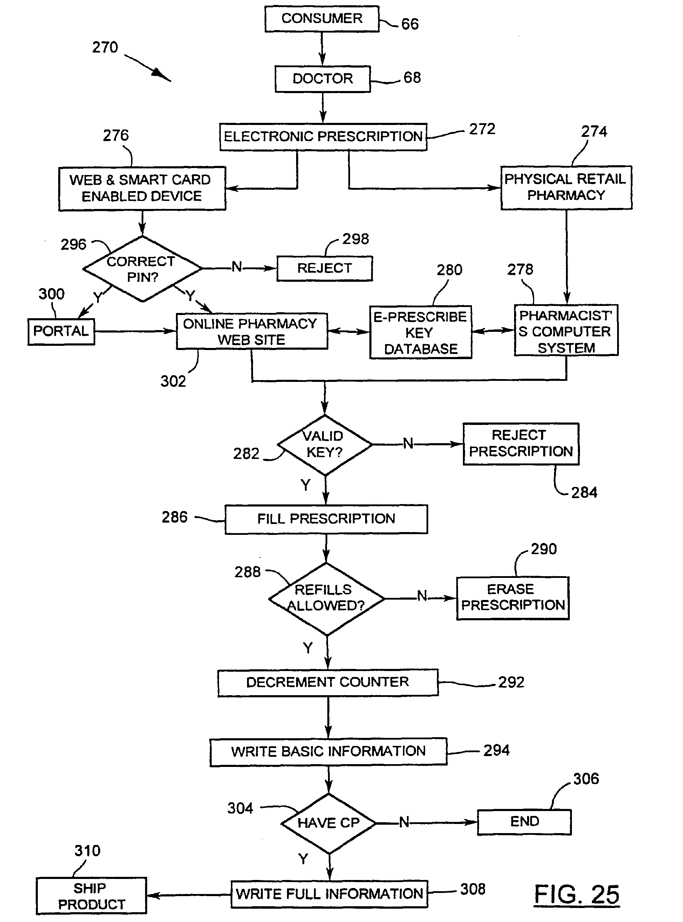

Referring to figure 25, the process of a consumer or patient purchasing on-line is shown generally at 270. The consumer or patient 66 attends at the physicians 68 and obtains an electronic prescription 272. To write the electronic prescription doctor places patient's smart health card (issued by either state/province, medical plan, or other organization) into the smartcard reader/writer attached to his computer and by using prescription writing software that is developed to be compatible with the system herein writes the prescription(s) onto the consumer's smart health card, along with his encrypted physician ID # and prescription authentication key (= e-prescribe key). In addition, preferably an incremented serial number is used (the pairing of the e-prescribe key with the incremented serial number creates a unique key for the individual prescription called for convenience the Prescription Unique Identifier). This relationship can be expressed as follows:

## e-prescribe key + serial # = PUI (Prescription Unique Identifier)

The consumer 66 can then either obtain the prescription from a retail outlet 274 or an on-line pharmacy 276.

If the consumer chooses to fill their prescription at a retail pharmacy 274, the consumer's smart health card with the prescription written thereon will be read by the pharmacy computer system 278 smartcard reader. The pharmacist's computer system reads e-prescribe key (including the PUI) and prescription from the consumer's smart health card. The pharmacy computer system 278 displays the prescription and searches the e-prescribe database 280 for a valid e-prescribe key 282.

If a match for the key is not found 284, the prescription is not authentic. If the match for the key is successful 286, the prescription is considered authentic and the order is filled.

The system then checks if repeats are allowed 288. If no, the prescription is erased 290 from the smart health card. Alternatively the system moves the prescription information to a memory location on the smartcard reserved for maintaining a recent history of medications purchased by the consumer, and erases the e-prescribe key. If yes, the repeats allowed counter on the smartcard is decremented 292 and the date, time and identity of the pharmacy filling the prescription is entered onto the smartcard. In addition, the system transfers the prescription information to the pharmacist's regular computer system software 294.

Alternatively the consumer 66 fills the prescription at an on-line pharmacy 276. The consumer inserts his smart health card into a smartcard reader. The software associated with the smart health card will prompt for a consumer personal identification number (PIN) 296. If the consumer PIN is incorrect 298 the person will not be able to purchase on-line with that smart health card.

If the consumer PIN is correct access to smartcard data is permitted. Once the smart health card has been validated the system can be configured in a number of ways. For example simultaneously, the system can automatically go on-line and launch a browser and go to a portal 300 or an on-line pharmacy web site 302 . Alternatively the user can navigate themselves. The consumer can set and reset the defaults such that on validating the consumer PIN the system goes to the site chosen by the consumer.

The portal site 300 can be configured to read the prescription from the smartcard and automatically searches registered on-line pharmacy sites for the prescribed medication and displays choices available (including generic drugs) and their prices. Alternatively the portal 300 could be an HMO or a health insurance site. In the event that the portal is either of these two organizations another level of validation may be included to facilitate payment of the prescription.

Once the pharmacy site 302 is chosen the consumer then indicates that he wants to fill the prescription. Thereafter the steps described above with regard to the on-line pharmacist's computer system reads e-prescribe key (including the PUI) and prescription from the consumer's smart health card. The on-line pharmacy computer system 302 displays the prescription and searches the e-prescribe database 280 for a valid e-prescribe key 282.

If a match for the key is not found 284, the prescription is not authentic. If the match for the key is successful 286, the prescription is considered authentic and the order is filled.

The system then checks if repeats are allowed 288. If no, the prescription is erased 290 from the smart health card. Alternatively the system moves the prescription information to a memory location on the smartcard reserved for maintaining a recent history of medications purchased by the consumer, and erases the e-prescribe key. If yes, the repeats allowed counter on the smartcard is decremented 292 and the date, time and identity of the pharmacy filling the prescription is entered onto the smartcard. In addition, the system transfers the prescription information to the pharmacist's regular computer system software 294.

In addition to the basic information being transferred to the pharmacist's computer system , whether retail or on-line, basic information is also written onto the label for the medication 294. Such information includes the name of medication and dosage/strength, number of pills (or some other measure of quantity) and frequency, number of repeats allowed, name of consumer, prescribing doctor, date issued, pharmacy's own transaction number. This information is put onto a conventional sticky label affixed to the medication bottle or package. In addition this information as well as an encrypted ID # that identifies the pharmacy and the PUI can be written onto an integrated circuit chip 14 to be affixed to the bottle or package with a smart label, or an integrated circuit chip integrally attached to a bottle or package as described above.

Further the system checks if the consumer has a consumer prompting device 304. If no 306, no further information is written onto the integrated circuit chip. If yes 308, further information is written onto the integrated circuit chip 14. Such information including warnings, contra-indications, corrective measures in the event of an error in self medication, encoded ranges used by the consumer prompting device to calculate the windows in time in which it will issue directions to the consumer to take a medication, encoded safe prescribing ranges used by the consumer prompting device to cross check prescribing information issued by the doctor, the PUI, a compressed image of the pill showing shape, markings and color (the universal method for identifying a pill) is written to the integrated circuit chip 14. Optionally the consumer could indicate a language preference during purchase, and information written to integrated circuit chip could be in that chosen language. Further it will be appreciated that the pharmacy may choose to include all this information even if the consumer indicates that they do not own a consumer prompting device.

Once all the information is written onto the label and the integrated circuit chip the product is ready to ship 310.

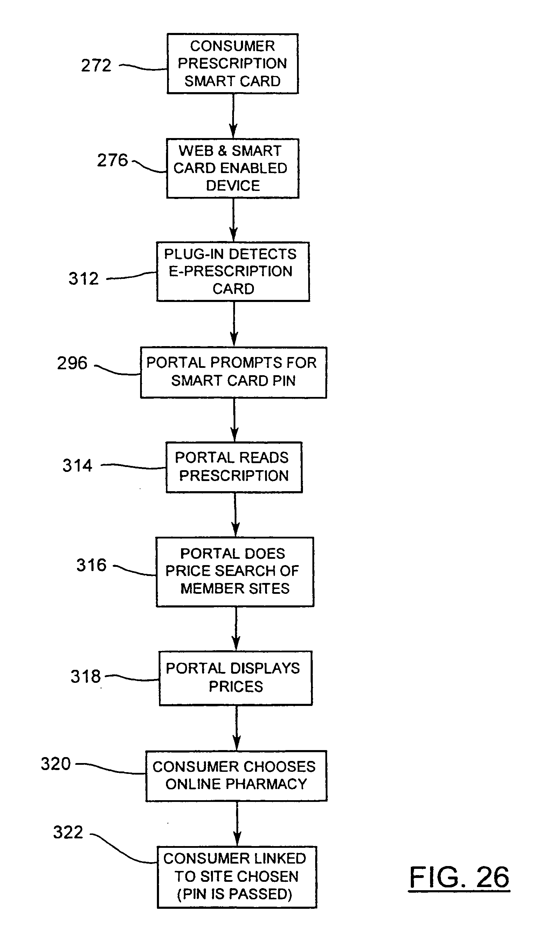

Referring to figure 26, as discussed above the consumer can set up his smart health card so that once it is read and validated the on-line connection is made directly to a portal. Specifically the consumer is given an electronic prescription 272 or the electronic prescription is written onto the consumer's smart health card. The smart health card is put into a smartcard reader or web and smartcard enabled device 276. The device detects that the card is present 312 and prompts for the consumer PIN 296. This consumer PIN verification may be done on the portal site or through the web and smartcard enabled device. Once on the portal site the prescription is read 314. The portal then searches 316 sites for product availability. Thereafter once the sites have been located the portal displays 318 the sites and the prices of the product at those sites. The consumer then chooses 320 from the sites where he wants to purchase the product and links 322 to that site. The consumer PIN can be passed to that site. Thereafter the prescription is filed as discussed above with regard to figure 25.

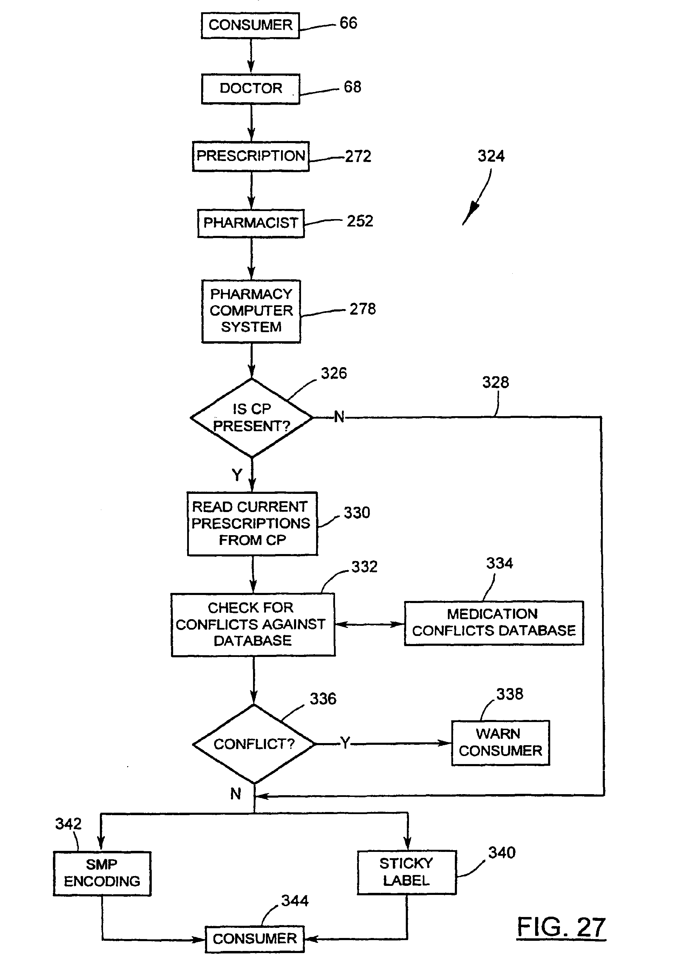

Referring to figure 27, the filing of a prescription can be adapted to use the consumer prompting device to determine if there are any conflicts. Since the consumer prompting device described above stores the names and the next take times of all the medication for which it prompts the consumer, it can be used to check for conflicts. The initial stages of the conflict checking system shown generally at 324 are similar to those when the consumer fills his prescription. The consumer or patient 66 attends at the physicians 68 and obtains an electronic prescription 272. The consumer 66 then goes to pharmacist 252, either an on-line pharmacist or a retail pharmacist. The system then prompts for a consumer prompting device 326. If there is no consumer prompting device present 328 a conflict check cannot be done using the consumer prompting device and the prescription is filled using the normal filling routines described above. Alternatively if the consumer prompting device is present the device is brought into proximity of an integrated circuit chip reader and the medications are read 330. If the consumer has his own integrated circuit chip reader connected to his web enabled device this conflict check could be done in conjunction with an on-line purchase. Alternatively it the consumer does not have such a reader this conflict check can be conducted at a pharmacy with such a reader. An example of such a reader is a Bluetooth™ or Jini™ enabled wireless reader. Once the information is read, the pharmacy computer system stores it in a temporary file. The new prescription is entered into the system and checks it for conflicts 332 against a medication conflicts database 334. If there is a conflict 336 the consumer is advised and consulted 338. In some circumstance this conflict may be acceptable, for example where it is acceptable to take the medications at an interval of a specified period of time. Alternatively it may be prudent to contact the physician and make alternate arrangements. If there are no conflicts the required information is written onto the sticky label 340 and information is written onto the integrated circuit chip 14 of the medication package 342 and the medication is given to the consumer 344.

Conflict checking system 324 could be adapted to be used in association with a smart health card rather than a consumer prompting device. Accordingly if the smart health card includes a list of all medications that the consumer is currently using that information can be read from the smart health card rather than the consumer prompting device.

Updates to the medication conflict database 334 are sent to all registered pharmacies. The database is sent via a secure socket layer document delivery web site (similar to DocSpace: www.docspace.com) or the like. Regular updates to the database are delivered (using the same technology) to each holder of the database. The downloaded update contains software to automatically revise and update the pharmacy's conflicts database and analysis software.

There are a number of advantages that can be realized by using automated prescription authentication. For example in the event that a physician retires or has his privileges removed a secure email can be sent to the pharmacists to update their e-prescribe key database and remove their authorization. Further the US Food and Drug Administration or other authorized body or watch dog organization can easily audit compliance with on-line prescription sales regulations by submitting a bogus transaction to a site and if the transaction is accepted, the site is not following regulations.

In addition, for the physician the system provides increased accuracy in both the recording and interpretation (by a pharmacy) of prescriptions issued by a physician (as compared to a hand written prescription). Further the system has the potential to allow a physician to positively track prescriptions he has issued and to reduce record keeping effort required of physician.

For the consumer the system provides an increased accuracy in reading a prescription which translates into greater safety for the consumer because there is much less risk of being issued the wrong medication. Further, by providing verifications at each step along the chain of filling a prescription at an on-line pharmacy there will be an increase in consumer confidence in regard to this method of filling a prescription. Still further, there will be an increase in the efficiency in utilizing on-line pharmacies whereby legitimate prescriptions can be verified and shipped immediately.

For the on-line pharmacy whether it be an independent or pharmaceutical products manufacturer, the system herein will facilitate faster and easier on-line purchases. Further the system herein will increase the pharmacy's protection from liability due to improved tracking and security features.

For the FDA or other regulatory or monitoring body the system herein provides effective tools for auditing compliance with on-line sales regulations.

Preferably the software that is used to implement the above described systems can be used on a wide variety of operating systems and platforms. A Java™ based system or a Java-type system would be portable and adaptable to pharmaceutical systems and hospital systems made by a number of manufacturers. Similarly where possible Jini™ technology or Jini-type technology would be used. These technologies enable all types of devices to simply connect into impromptu networks, making access to and delivery of new network services as simple as plugging in a telephone. Built on top of a Java™ software infrastructure, Jini™ technology enables all types of digital devices to work together in a community put together without extensive planning, installation, or human intervention. Each device provides services that other devices in the community may use. These devices provide their own user or programmatic interfaces, which ensures reliability and compatibility. Bluetooth is a technology specification for lowcost, short range radio links between PDAs, laptops, mobile phones, and other portable devices. Preferably, this type of technology would be used with the consumer prompting device, readers for the consumer prompting device and readers for the smart health card. When two Bluetooth™ devices come close together, they automatically detect each other and establish a network connection. This is an example of a network transport protocol that could be used to allow devices using Jini™ technology to communicate without being physically connected to each other. Other technology like Piano™, which can be built on top of Bluetooth™, specifies what sort of information they exchange and how they communicate. It and other operating systems, like EPOC32™ for cell phones provide the necessary features to support Jini™ technology.

It will be appreciated by those skilled in the art that the consumer prompting device of the present invention may be used as a single purpose device. For example it could be adapted for use in association with the birth control pill. Since in this example only one medication is being monitored the systems can be greatly simplified. Alternatively the consumer prompting device of the present invention could be used as a multi-person prompting system for example, for a family. In this embodiment the reminder notification would identify not only the medication but also the user. The different users could also be identified with a different sound. Each user could have a unique identifier for their own use so that not only the medication could be verified but also the user.

There are a number of advantages of the present invention over the prior art. For example since the information with regard to the parameters for determining the next take time, best practices, contra indicators, hazards and the like is stored on the individual medication packages the consumer prompter device 20, 45 can manage a large number of medications because the only information that is required to be stored on device 20, 45 is the name of the medication, the next take time and the take window. Alternatively where the memory available on the integrated circuit chip is low and certain indexes would also be stored on device 20, 45 the consumer prompter can still manage a large number of medications. Accordingly, the memory requirements for each medication are relatively low. Further, the cost of attaching an integrated circuit chip to a package is relatively low and since no power source is required in association with the package 10 (only device 20), the cost of operating the prompting and monitoring system of the present invention is relatively low. Since the integrated circuit chip 14 is non-volatile it need not be attached to device 20 to store information and thus device 20 can readily be used with multiple medications. The present invention provides a simple, user friendly method for monitoring the correct use of the consumer's medication. The present invention provides a method of prompting the consumer to take a medication and then verifying that it is the correct medication before the consumer takes the particular medication. As discussed above, after the consumer prompting device 20 prompts the consumer to take a medication, the integrated circuit chip 14 identified as being associated with the particular medication must be read by the read/write module so as to confirm that the correct medication was taken. Thus the consumer prompting device provides a method of verifying that the correct medication was taken.

A further advantage of the present system is that the times at which the medication is actually taken is stored on the integrated circuit chip 14 thus the physician or other health care professional can monitor the actual use of the medication. This information can be invaluable when considering altering the dosage or use of the medication. Further, this information will be useful for medical and pharmaceutical research and in some instances for the patient's health insurance.

It will be appreciated that the above description relates to the invention by way of example only. Many variations on the invention will be obvious to those skilled in the art and such obvious variations are within the scope of the invention as described herein whether or not expressly described.

**Claims**

1. An interactive reminder device (20) to prompt a patient to take medication stored in a package (10) in a timely manner, the device comprising means for storing a next take time for the medication and means for prompting when the next take time is due, the device:

   (a) storing a unique identifier (84);
   (b) being able to write (30) that unique identifier to an integrated circuit chip (14) on the medication package (10) if no such unique identifier is already present on the integrated circuit (14); and
   (c) being able to read (30) an identifier (84) from an integrated circuit chip (14) on a medication package (10) presented to the device and being able to compare (100) it to the stored unique identifier and to confirm to the patient (106) that the package contains medication for that patient only if there is a match (102).

2. An interactive reminder device as claimed in claim 1 able to prompt the patient to take medication using one or more of a beep (42), a vibration (41) or a visual indicator (28).

3. An interactive reminder device as claimed in any previous claim wherein the device is incorporated in a device personal to the patient, the device being selected from the group of: an electronic personal organizer, a cell phone, a personal digital assistant and a hand held computer.

4. An interactive reminder device as claimed in any previous claim which can prompt the patient to take a specific medication and can then determine (130, 132) if the medication package presented to it is in fact that specific medication.

5. An interactive reminder device as claimed in any previous claim which a patient can present a medication package to and the device can then determine (122) if the medication should in fact be taken by the patient at that time.

6. An interactive reminder device as claimed in any previous claim which can write (96) the next take time for a particular medication onto an integrated circuit chip on the medication package.

7. An interactive reminder device as claimed in any previous claim which can read the parameters for calculating the next take time for a particular medication from an integrated circuit chip (14) on the medication package (10) and then work out the next take time, and store that in device memory (43).

8. An interactive reminder device as claimed in any previous claim which can store (43) a record of the times at which the patient took different medications.

9. An interactive reminder device as claimed in claim 8 which can send the record of the times at which the patient took different medications to a remote computer programmed to monitor the use of medication.

10. A method of prompting a patient to take medication stored in a medication package in a timely manner, the method comprising the steps of:

(a) storing a unique identifier in an interactive reminder device (20);
(b) the device writing (30) that unique identifier to an integrated circuit chip (14) on the medication package if no such unique identifier is already present on the integrated circuit;
(c) the device storing a next take time for the medication and prompting (122) when the next take time is due;
(d) the device reading an identifier from an integrated circuit chip on a medication package presented (128) to the device and comparing it (130) to the stored unique identifier;
(e) the device confirming to the patient (136) that the package contains medication for that patient only if there is a match.

11. A method for prompting for the use of medication as claimed in claim 10 further including the steps of the device (20) identifying an identifiable integrated circuit chip (14) associated with a stored next take time and confirming the identifiable integrated circuit chip (14) is associated with a prompted next take time.

12. A method for prompting for the use of medication as claimed in any of claims 10 and 11 wherein a name of the medication associated with the integrated circuit chip is stored in association with the next take time on the device and the name is displayed (106) on the device at the next take time.

13. A method for prompting for the use of medication as claimed in any of claims 10 to 12 further including the steps of the device identifying that the medication is taken and writing (40) the take time onto the integrated circuit chip (14) attached to the medication package.

14. A method for prompting for the use of medication as claimed in any of claims 10 to 13 further including the steps of the device identifying that the medication is taken and writing (40) the take time onto the device.

15. A method for prompting for the use of medication as claimed in any of claims 10 to 14 further including the steps of the device identifying that the medication is taken and writing the take time onto an integrated circuit chip in a smartcard.

16. A method for prompting for the use of medication as claimed in any of claims 10 to 15 further including the steps of the device calculating a next take time window and identifying if the present time is within the next take window.

**17.** A method for prompting for the use of medication as claimed in any of claims 10 to 16 further including the steps of the device calculating a deferred next take time and prompting at the deferred next take time.

**18.** A method for prompting for the use of medication as claimed in any of claims 10 to 17 further including the step of the device reading and displaying further information stored on the integrated circuit chip attached to the medication package.

**19.** A method for prompting for the use of medication as claimed in claim 18 wherein the parameters and the further information are stored as alphanumeric codes and the alphanumeric codes correspond to specific information.

**20.** A method for monitoring the use of medication in a health care facility comprising the steps of:

(a) prompting a patient to take medication stored in a medication package in a timely manner as defined in Claim 10 and
(b) receiving information from the personal interactive reminder device defining how the patient has taken his medication.

**21.** The method of Clam 20 comprising the further step of prompting another patient to take medication stored in a medication package in a timely manner as defined in Claim 10 and receiving information from the personal interactive reminder device defining how this other patient has taken his medication

**22.** A medication package (10) when used with the interactive reminder device (20) of Claim 1, the package comprising an integrated circuit chip (14) that can have written to it by the interactive reminder device (20) a unique identifier that associates the package (10) with the interactive reminder device (20).

**Patentansprüche**

**1.** Interaktive Erinnerungsvorrichtung (20), um einen Patienten rechtzeitig dazu aufzufordern, Medizin einzunehmen, die in einer Verpackung (10) gelagert ist, wobei die Vorrichtung Mittel zum Speichern eines nächsten Zeitpunkts zur Einnahme der Medizin und Mittel zum Auffordern, wann der nächste Zeitpunkt ist, umfasst, wobei die Vorrichtung:

(a) einen eindeutigen Kennzeichner (84) speichert;
(b) diesen eindeutigen Kennzeichner in einen Chip einer integrierten Schaltung (14) auf der Medizinverpackung (10) schreiben (30) kann, falls kein derartiger eindeutiger Kennzeichner bereits auf der integrierten Schaltung (14) vorhanden ist; und
(c) einen Kennzeichner (84) von einem Chip einer integrierten Schaltung (14) auf einer Medizinverpackung (10), die der Vorrichtung vorgelegt wird, lesen (30) kann und diesen mit dem gespeicherten eindeutigen Kennzeichner vergleichen (100) kann und dem Patienten (106) bestätigen kann, dass die Verpackung Medizin für diesen Patienten enthält, nur wenn eine Übereinstimmung (102) vorliegt.

**2.** Interaktive Erinnerungsvorrichtung nach Anspruch 1, die den Patienten dazu auffordern kann, Medizin einzunehmen, indem sie einen oder mehrere von einem Piepston (42), einer Vibration (41) oder ein visuelles Anzeigeelement (28) benutzt.

**3.** Interaktive Erinnerungsvorrichtung nach einem der vorhergehenden Ansprüche, wobei die Vorrichtung in einer persönlichen Vorrichtung des Patienten eingebaut ist, wobei die Vorrichtung aus folgender Gruppe ausgewählt ist: einem elektronischen persönlichen Notizbuch, einem Mobiltelefon, einem PDA und einem Handcomputer.

**4.** Interaktive Erinnerungsvorrichtung nach einem der vorhergehenden Ansprüche, die den Patienten dazu auffordern kann, eine spezifische Medizin einzunehmen und dann bestimmen (130, 132) kann, ob die ihr vorgelegte Medizinverpackung tatsächlich die spezifische Medizin ist.

**5.** Interaktive Erinnerungsvorrichtung nach einem der vorhergehenden Ansprüche, der ein Patient eine Medizinverpackung vorlegen kann und die Vorrichtung dann bestimmen (122) kann, ob die Medizin tatsächlich vom Patienten zu diesem Zeitpunkt eingenommen werden soll.

**6.** Interaktive Erinnerungsvorrichtung nach einem der vorhergehenden Ansprüche, welche den nächsten Zeitpunkt für eine bestimmte Medizin auf einen Chip einer integrierten Schaltung auf der Medizinverpackung schreiben (96) kann.

7. Interaktive Erinnerungsvorrichtung nach einem der vorhergehenden Ansprüche, welche die Parameter zur Berechnung des nächsten Zeitpunkts für eine bestimmte Medizin von einem Chip einer integrierten Schaltung (14) auf der Medizinverpackung (10) lesen kann und dann den nächsten Zeitpunkt zur Einnahme ermitteln kann und dies im Speicher (43) der Vorrichtung speichern kann.

8. Interaktive Erinnerungsvorrichtung nach einem der vorhergehenden Ansprüche, welche eine Aufzeichnung der Zeitpunkte, zu denen der Patient unterschiedliche Medikamente einnahm, speichern (43) kann.

9. Interaktive Erinnerungsvorrichtung nach Anspruch 8, welche die Aufzeichnung der Zeitpunkte, zu denen der Patient unterschiedliche Medikamente einnahm, einem entfernten Computer senden kann, der zur Überwachung der Verwendung der Medizin programmiert ist.

10. Verfahren zur Aufforderung eines Patienten zur rechtzeitigen Einnahme von Medizin, die in einer Medizinverpackung gelagert ist, wobei das Verfahren die folgenden Schritte umfasst:

    (a) Speichern eines eindeutigen Kennzeichners in einer interaktiven Erinnerungsvorrichtung (20);
    (b) Schreiben (30) dieses eindeutigen Kennzeichners in einen Chip einer integrierten Schaltung (14) auf der Medizinverpackung durch die Vorrichtung, falls kein derartiger eindeutiger Kennzeichner bereits auf der integrierten Schaltung vorhanden ist;
    (c) Speichern eines nächsten Zeitpunkts zur Einnahme der Medizin und Auffordern (122), wann der nächste Zeitpunkt ist, durch die Vorrichtung;
    (d) Lesen eines Kennzeichners aus einem Chip einer integrierten Schaltung auf einer Medizinverpackung durch die Vorrichtung, welche der Vorrichtung vorgelegt (128) wird, und Vergleichen (130) dieser mit dem eindeutigen Kennzeichner;
    (e) Bestätigen gegenüber dem Patienten (136) durch die Vorrichtung, dass die Verpackung Medizin für diesen Patienten enthält, nur wenn eine Übereinstimmung vorliegt.

11. Verfahren zur Aufforderung zur Benutzung von Medizin nach Anspruch 10, ferner umfassend die Schritte, dass die Vorrichtung (20) einen identifizierbaren Chip einer integrierten Schaltung (14), die mit einem gespeicherten nächsten Zeitpunkt zur Einnahme in Verbindung steht, identifiziert und bestätigt, dass der identifizierbare Chip der integrierten Schaltung (14) mit einem aufgeforderten nächsten Zeitpunkt zur Einnahme in Verbindung steht.

12. Verfahren zur Aufforderung zur Benutzung von Medizin nach einem der Ansprüche 10 und 11, wobei ein Name der Medizin, die mit dem Chip der integrierten Schaltung in Verbindung steht, in Verbindung mit dem nächsten Zeitpunkt zur Einnahme auf der Vorrichtung gespeichert ist und der Name zum nächsten Zeitpunkt zur Einnahme auf der Vorrichtung angezeigt (106) wird.

13. Verfahren zur Aufforderung zur Benutzung von Medizin nach einem der Ansprüche 10 bis 12, ferner umfassend die Schritte, dass die Vorrichtung identifiziert, dass die Medizin eingenommen wird, und den Zeitpunkt zur Einnahme auf den Chip der integrierten Schaltung (14), welche an der Medizinverpackung befestigt ist, schreibt (40).

14. Verfahren zur Aufforderung zur Benutzung von Medizin nach einem der Ansprüche 10 bis 13, ferner umfassend die Schritte, dass die Vorrichtung identifiziert, dass die Medizin eingenommen wird, und den Zeitpunkt zur Einnahme auf die Vorrichtung schreibt (40).

15. Verfahren zur Aufforderung zur Benutzung von Medizin nach einem der Ansprüche 10 bis 14, ferner umfassend die Schritte, dass die Vorrichtung identifiziert, dass die Medizin eingenommen wird, und den Zeitpunkt zur Einnahme auf einen Chip einer integrierten Schaltung in einer Chipkarte schreibt.

16. Verfahren zur Aufforderung zur Benutzung von Medizin nach einem der Ansprüche 10 bis 15, ferner umfassend die Schritte, dass die Vorrichtung ein nächstes Intervall für den nächsten Zeitpunkt zur Einnahme berechnet und identifiziert, ob die Jetztzeit innerhalb des Intervalls für den nächsten Zeitpunkt zur Einnahme liegt.

17. Verfahren zur Aufforderung zur Benutzung von Medizin nach einem der Ansprüche 10 bis 16, ferner umfassend die Schritte, dass die Vorrichtung einen verschobenen nächsten Zeitpunkt zur Einnahme berechnet und zum verschobenen nächsten Zeitpunkt zur Einnahme auffordert.

**18.** Verfahren zur Aufforderung zur Benutzung von Medizin nach einem der Ansprüche 10 bis 17, ferner umfassend den Schritt, dass die Vorrichtung weitere Informationen, die im Chip der integrierten Schaltung gespeichert sind, welche an der Medizinverpackung befestigt ist, liest und anzeigt.

**19.** Verfahren zur Aufforderung zur Benutzung von Medizin nach Anspruch 18, wobei die Parameter und die weiteren Informationen als alphanumerische Codes gespeichert sind und die alphanumerischen Codes spezifischen Informationen entsprechen.

**20.** Verfahren zur Überwachung der Benutzung von Medizin in einer Gesundheitsfürsorgeeinrichtung, umfassend die Schritte:

(a) Auffordern eines Patienten zur rechtzeitigen Einnahme von Medizin, die in einer Medizinverpackung gelagert ist, nach Anspruch 10 und
(b) Empfangen von Informationen von einer persönlichen interaktiven Erinnerungsvorrichtung, wobei definiert wird, wie der Patient seine Medizin eingenommen hat.

**21.** Verfahren nach Anspruch 20, umfassend den weiteren Schritt zum Auffordern eines anderen Patienten zur rechtzeitigen Einnahme von Medizin, die in einer Medizinverpackung gelagert ist, nach Anspruch 10, und Empfangen von Informationen von der persönlichen interaktiven Erinnerungsvorrichtung, wobei definiert wird, wie dieser andere Patient seine Medizin eingenommen hat.

**22.** Medizinverpackung (10), wenn mit der interaktiven Erinnerungsvorrichtung (20) von Anspruch 1 benutzt, wobei die Verpackung einen Chip einer integrierten Schaltung (14) umfasst, auf den durch die interaktive Erinnerungsvorrichtung (20) ein eindeutiger Kennzeichner geschrieben werden kann, welcher die Verpackung (10) mit der interaktiven Erinnerungsvorrichtung (20) in Verbindung bringt.

**Revendications**

**1.** Dispositif de rappel interactif (20) pour rappeler à un patient de prendre des médicaments stockés dans un paquet (10) de façon opportune, le dispositif comprenant un moyen pour mémoriser un prochain moment de prise des médicaments et un moyen pour émettre un rappel lorsque le prochain moment de prise arrive, le dispositif :

(a) mémorisant un identifiant unique (84);
(b) étant capable d'inscrire (30) cet identifiant unique sur une puce de circuit intégré (14) sur le paquet de médicaments (10) si aucun identifiant unique de la sorte n'est déjà présent sur le circuit intégré (14) ; et
(c) étant capable de lire (30) un identifiant (84) à partir d'une puce de circuit intégré (14) sur un paquet de médicaments (10) présenté au dispositif et étant capable de le comparer (100) à l'identifiant unique mémorisé et de confirmer au patient (106) que le paquet contient des médicaments pour ce patient uniquement en cas de correspondance (102).

**2.** Dispositif de rappel interactif tel que revendiqué dans la revendication 1, capable de rappeler au patient de prendre des médicaments à l'aide d'un ou plusieurs éléments parmi un bip (42), une vibration (41) ou un indicateur visuel (28).

**3.** Dispositif de rappel interactif tel que revendiqué dans n'importe quelle revendication précédente dans lequel le dispositif est incorporé dans un dispositif personnel au patient, le dispositif étant sélectionné dans le groupe composé de : un organiseur personnel électronique, un téléphone cellulaire, un assistant numérique personnel et un ordinateur de poche.

**4.** Dispositif de rappel interactif tel que revendiqué dans n'importe quelle revendication précédente qui peut rappeler au patient de prendre un médicament spécifique et peut ensuite déterminer (130, 132) si le paquet de médicaments qui lui est présenté est en réalité ce médicament spécifique.

**5.** Dispositif de rappel interactif tel que revendiqué dans n'importe quelle revendication précédente auquel un patient peut présenter un paquet de médicaments, le dispositif pouvant ensuite déterminer (122) si le patient doit en réalité prendre le médicament à ce moment.

**6.** Dispositif de rappel interactif tel que revendiqué dans n'importe quelle revendication précédente qui peut inscrire (96) le prochain moment de prise d'un médicament particulier sur une puce de circuit intégré sur le paquet de médicaments.

7. Dispositif de rappel interactif tel que revendiqué dans n'importe quelle revendication précédente qui peut lire les paramètres pour calculer le prochain moment de prise pour un médicament particulier à partir d'une puce de circuit intégré (14) sur le paquet de médicaments (10) et calculer ensuite le prochain moment de prise, et le mémoriser dans la mémoire de dispositif (43).

8. Dispositif de rappel interactif tel que revendiqué dans n'importe quelle revendication précédente qui peut mémoriser (43) un enregistrement des moments auxquels le patient a pris différents médicaments.

9. Dispositif de rappel interactif tel que revendiqué dans la revendication 8 qui peut envoyer l'enregistrement des moments auxquels le patient a pris différents médicaments à un ordinateur distant programmé pour contrôler l'utilisation des médicaments.

10. Méthode de rappel à un patient de prendre des médicaments stockés dans un paquet de médicaments de façon opportune, la méthode comprenant les étapes de :

   (a) mémoriser un identifiant unique dans un dispositif de rappel interactif (20) ;
   (b) le dispositif inscrivant (30) cet identifiant unique sur une puce de circuit intégré (14) sur le paquet de médicaments si aucun identifiant unique de la sorte n'est déjà présent sur le circuit intégré ;
   (c) le dispositif mémorisant un prochain moment de prise des médicaments et émettant un rappel (122) lorsque le prochain moment de prise est arrivé ;
   (d) le dispositif lisant un identifiant à partir d'une puce de circuit intégré sur un paquet de médicaments présenté (128) au dispositif et le comparant (130) à l'identifiant unique mémorisé ;
   (e) le dispositif confirmant au patient (136) que le paquet contient les médicaments pour ce patient uniquement en cas de correspondance.

11. Méthode de rappel pour l'utilisation de médicaments telle que revendiquée dans la revendication 10 incluant de plus les étapes du dispositif (20) identifiant une puce de circuit intégré identifiable (14) associée à un prochain moment de prise mémorisé et confirmant que la puce de circuit intégré identifiable (14) est associée à un prochain moment de prise rappelé.

12. Méthode de rappel pour l'utilisation de médicaments telle que revendiquée dans n'importe lesquelles des revendications 10 et 11 dans laquelle un nom du médicament associé à la puce de circuit intégré est mémorisé en association avec le prochain moment de prise sur le dispositif et le nom est affiché (106) sur le dispositif au prochain moment de prise.

13. Méthode de rappel pour l'utilisation de médicaments telle que revendiquée dans n'importe lesquelles des revendications 10 à 12 incluant de plus les étapes du dispositif identifiant que le médicament est pris et inscrivant (40) l'heure de prise sur la puce de circuit intégré (14) attachée au paquet de médicaments.

14. Méthode de rappel pour l'utilisation de médicaments telle que revendiquée dans n'importe lesquelles des revendications 10 à 13 incluant de plus les étapes du dispositif identifiant que le médicament est pris et inscrivant (40) l'heure de prise sur le dispositif.

15. Méthode de rappel pour l'utilisation de médicaments telle que revendiquée dans n'importe lesquelles des revendications 10 à 14 incluant de plus les étapes du dispositif identifiant que le médicament est pris et inscrivant l'heure de prise sur une puce de circuit intégré dans une carte intelligente.

16. Méthode de rappel pour l'utilisation de médicaments telle que revendiquée dans n'importe lesquelles des revendications 10 à 15 incluant de plus les étapes du dispositif calculant une prochaine fenêtre de temps de prise et identifiant si le moment présent est compris dans la prochaine fenêtre de prise.

17. Méthode de 'rappel pour l'utilisation de médicaments telle que revendiquée dans n'importe lesquelles des revendications 10 à 16 incluant de plus les étapes du dispositif calculant un prochain moment de prise différé et émettant un rappel au prochain moment de prise différé.

**18.** Méthode de rappel pour l'utilisation de médicaments telle que revendiquée dans n'importe lesquelles des revendications 10 à 17 incluant de plus les étapes du dispositif lisant et affichant des informations supplémentaires mémorisées sur la puce de circuit intégré attachée au paquet de médicaments.

**19.** Méthode de rappel pour l'utilisation de médicaments telle que revendiquée dans la revendication 18 dans laquelle les paramètres et les informations supplémentaires sont mémorisés sous forme de codes alphanumériques et les codes alphanumériques correspondent à des informations spécifiques.

**20.** Méthode pour contrôler l'utilisation de médicaments dans un établissement de santé comprenant les étapes de :

(a) rappeler à un patient de prendre des médicaments stockés dans un paquet de médicaments de façon opportune telle que définie dans la revendication 10 et
(b) recevoir des informations provenant du dispositif de rappel interactif personnel définissant la manière dont le patient a pris ses médicaments.

**21.** Méthode de la revendication 20 comprenant l'étape supplémentaire de rappeler à un autre patient de prendre des médicaments stockés dans un paquet de médicaments de façon opportune telle que définie dans la revendication 10 et recevoir des informations provenant du dispositif de rappel interactif personnel définissant la manière dont cet autre patient a pris ses médicaments.

**22.** Paquet de médicaments (10) lorsqu'utilisé avec le dispositif de rappel interactif (20) de la revendication 1, le paquet comprenant une puce de circuit intégré (14) qui peut avoir un identifiant unique qui associe le paquet (10) au dispositif de rappel , interactif (20), l'identifiant unique étant inscrit par le dispositif de rappel interactif (20).

FIG. 1

FIG. 2

FIG. 3

FIG. 4

6

20

37    36

37

32

FIG.5

20

7    7

36    208  37    37  208    32

FIG. 6

FIG. 10

FIG. 7

200

202

14

206

204

FIG. 8

14 202

200

FIG. 9

200

20

32

FIG. 11

FIG. 13

FIG. 12

FIG. 14

FIG. 15

FIG. 16

FIG. 17

FIG. 18

CONSUMER — 66

64

DOCTOR — 68

PRESCRIPTION — 70

PHARMACIST — 72

PHARMACY COMPUTER SYSTEM — 74

78 — SMP ENCODING

STICKY LABEL — 76

CONSUMER — 66

FIG. 19

CONSUMER —66

INSERT —82

READ IDENTIFIER —84

80

88— DISPLAY INFORMATION ← Y — EMPTY? —86 — N → COMPARE —100

94 — PRESS "TAKE" BUTTON ← Y — TAKE NOW? —90 — N → "REMOVE" —92

102 — SAME?

106 — DISPLAY INFORMATION

104 — "NOT YOURS"

EXCHANGE INFORMATION —96

"REMOVE" —98

FIG. 20

FIG.21

EP 1 210 052 B1

68
HOSPITAL DOCTOR

70
PRESCRIPTION

150
FLOOR NURSING STATION STAFF

154

152

162

166
Insert Norvasc
#245634
Patient ID 893848
Room 8K

164

170   168

148

156
READER/WRITER

CUSTOM SMP READER/WRITER ATTACHMENT TO PALM PILOT OR SIMILAR DEVICE

NURSE'S PROMPTER (NP)

158

14

160

FIG. 22

EP 1 210 052 B1

37

DOCTOR — 68

230

234 — REGISTRATION WEB SITE ⟷ 236 — GOVERNMENT CONTOLLED AUTHENTICATION DATABASE

238 — REGISTRATION SUCCESSFULL?

N → 240 — NOTIFY DOCTOR OF REJECTION

Y

242 — ACTIVATE ACCOUNT

<u>FIG. 23</u>

PHARMACIST —— 252

250

256

254 —

REGISTRATION
WEB SITE

GOVERNMENT CONTOLLED
AUTHENTICATION DATABASE

258

260

REGISTRATION
SUCCESSFUL?

—N—→

NOTIFY PHARMACIST
OF REJECTION

Y

262

ACTIVATE
ACCOUNT

FIG. 24

FIG. 25

272 → CONSUMER PRESCRIPTION SMART CARD

276 → WEB & SMART CARD ENABLED DEVICE

312 → PLUG-IN DETECTS E-PRESCRIPTION CARD

296 → PORTAL PROMPTS FOR SMART CARD PIN

314 → PORTAL READS PRESCRIPTION

316 → PORTAL DOES PRICE SEARCH OF MEMBER SITES

318 → PORTAL DISPLAYS PRICES

320 → CONSUMER CHOOSES ONLINE PHARMACY

322 → CONSUMER LINKED TO SITE CHOSEN (PIN IS PASSED)

FIG. 26

FIG. 27

**EP 1 210 052 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4616316 A
- US 5181189 A, Hafner
- US 5495961 A, Maestre
- US 4504153 A, Schollmeyer
- US 4831562 A, McIntosh
- US 5805051 A, Hermann
- US 5812064 A, Barbour
- US 5802015 A, Rothschild
- US 4695954 A
- US 5852590 A